Europäisches Patentamt

European Patent Office

Office européen des brevets

(19)

(11)  EP 1 562 149 A2

(12)  # EUROPEAN PATENT APPLICATION

(43) Date of publication:
    10.08.2005  Bulletin 2005/32

(51) Int Cl.⁷: $G06T\ 11/00$

(21) Application number: 05250549.2

(22) Date of filing: 01.02.2005

(84) Designated Contracting States:
    AT BE BG CH CY CZ DE DK EE ES FI FR GB GR
    HU IE IS IT LI LT LU MC NL PL PT RO SE SI SK TR
    Designated Extension States:
    AL BA HR LV MK YU

(30) Priority: 06.02.2004 JP 2004030727

(71) Applicant: GE Medical Systems Global
    Technology Company LLC
    Waukesha, Wisconsin 53188-1696 (US)

(72) Inventors:
    • Horiuchi, Tetsuya
      Hino-shi, Tokyo 191-8503 (JP)
    • Morikawa, Kotoko
      Hino-shi, Tokyo 191-8503 (JP)

(74) Representative: Goode, Ian Roy
    London Patent Operation
    General Electric International, Inc.
    15 John Adam Street
    London WC2N 6LU (GB)

(54)  **Image reconstructing method and X-ray CT apparatus**

(57)    The present invention aims to provide an image reconstructing method which obtains an image good in quality where a helical scan with a scan surface being tiled is performed using a cone beam. A tilt correcting process for correcting variations every views, in positions of respective channels of detector sequences with respect to a linear travel axis due to the inclination of the scan surface is effected on projection data D0. Data D1 plane-projected onto a projection plane are determined on the basis of the tilt-corrected projection data D0 (R1). Next, the plane-projected data D1 are projected, in an X-ray penetration direction, onto respective pixels constituting a plurality of lines which are placed on a reconstruction area with plural pixel intervals defined thereamong and extend in the direction parallel to the projection plane to thereby determine backprojected pixel data D2 of respective pixels constituting the lines on the reconstruction area. Further, interpolation is applied among the plurality of lines to determine backprojected pixel data D2 of respective pixels among the lines on the reconstruction area (R2). The backprojected pixel data D2 of all views used in image reconstruction are added together in association with the pixels (R3).

Fig. 1 6

EP 1 562 149 A2

EP 1 562 149 A2

**Description**

The present invention relates to an image reconstructing method and an X-ray CT (Computed Tomography) apparatus, and particularly to a method of reconstructing an image on the basis of projection data obtained by a helical scan with a tilted scan surface, using a multi-row detector, and an X-ray CT apparatus therefor.

In an X-ray CT apparatus, a helical scan with a scan surface tilted is conventionally effective as a method for imaging a depressed bareface of a subject in avoidance of exposure of its crystalline lens to radiation. When projection data are collected by the helical scan with the tilted scan surface through the use of a multi-row detector, and an image is reconstructed based on it, a tilt correction is effected on the projection data and image reconstruction is carried out using the tilt-corrected projection data (refer to, for example, Japanese Unexamined Patent Publication No. 2003-61948 (7th to 8th pages and Figs. 6 through 14)).

The more the number of sequences of a multi-row detector increases, the more the X ray becomes pronounced in the property of a cone beam. Therefore, a contradiction between projection data related to an image reconstruction area cannot be ignored. Thus, it is not possible to prevent archfacts from occurring in a reconstructed image under the utilization of a tilt correction alone.

Therefore, an object of the present invention is to realize an image reconstructing method which obtains an image good in quality where a helical scan with a scan surface being tilted is performed using a cone beam, and an X-ray CT apparatus therefor.

According to a first aspect, the present invention provides an image reconstructing method comprising the steps of, upon reconstructing an image using a multi-row detector having a plurality of detector sequences and on the basis of projection data D0 collected by a helical scan with a scan surface being tilted: effecting a tilt correcting process for correcting variations every views, in positions of respective channels of the detector sequences with respect to a linear travel axis due to the inclination of the scan surface, on the projection data D0; then projecting respective pixels in an X-ray penetration direction along lines parallel to an X axis or a Y axis on a reconstruction plane (XY plane) to determine the corresponding projection data D0 and defining the same as backprojected pixel data D2 of respective pixels constituting the reconstruction plane; and adding the backprojected pixel data D2 of all views used in image reconstruction in association with the pixels to determine backprojected data D3.

According to a second aspect, the present invention provides an image reconstructing method wherein the projection data D0 are fan-para converted from projection data D0f corresponding to collected fan data to projection data D0p corresponding to parallel data in advance and are based on the projection data D0p corresponding to the parallel data.

According to a third aspect, the present invention provides an image reconstructing method comprising the steps of, upon reconstructing an image using a multi-row detector having a plurality of detector sequences and on the basis of projection data D0 collected by a helical scan with a scan surface being tilted: effecting a tilt correcting process for correcting variations every views, in positions of respective channels of the detector sequences with respect to a linear travel axis due to the inclination of the scan surface, on the projection data D0; determining data D1 plane-projected onto the plane of projection on the basis of the tilt-corrected projection data D0; then projecting, in an X-ray penetration direction, the data D1 plane-projected on respective pixels constituting a plurality of lines which are placed on a reconstruction area with plural pixel intervals defined thereamong and extend in the direction parallel to the projection plane to thereby determine backprojected pixel data D2 of the respective pixels constituting the lines on the reconstruction area; and interpolating among the plurality of lines to determine backprojected pixel data D2 of respective pixels among the lines on the reconstruction area; and adding the backprojected pixel data D2 of all views employed in image reconstruction in association with the pixels to determine backprojected data D3.

According to a fourth aspect, the present invention provides an image reconstructing method 5comprising the steps of, upon reconstructing an image using a multi-row detector having a plurality of detector sequences and on the basis of projection data D0 collected by a helical scan with a scan surface being tilted: effecting a tilt correcting process for correcting variations every views, in positions of respective channels of the detector sequences with respect to a linear travel axis due to the inclination of the scan surface, on the projection data D0; determining data D1 plane-projected onto lines on a projection plane, corresponding to a plurality of lines which are placed on a reconstruction area with plural pixel intervals defined thereamong and extend in the direction parallel to the projection plane, on the basis of the tilt-corrected projection data D0; determining backprojected pixel data D2 of respective pixels on the reconstruction area on the basis of the plane-projected data D1 located on the lines on the projection plane; and adding the backprojected pixel data D2 of all views used in image reconstruction in association with the pixels to determine backprojected data D3.

According to a fifth aspect, the present invention provide an image reconstructing method wherein in the image reconstructing method having the above configuration, the number of the lines ranges from 1/64 to 1/2 of the number of the pixels in the reconstruction area as viewed in the direction orthogonal to the lines.

According to a sixth aspect, the present invention provides an image reconstructing method wherein in the image reconstructing method having the above configuration, when the direction orthogonal to a rotating plane of an X-ray tube or the multi-row detector is defined as a z direction, the direction of a center axis of an X-ray beam at view = 0° is defined as a y direction, and the direction orthogonal to the z direction and the y direction is defined as an x direction, an xz plane passing through the center of rotation is configured as the projection plane in -45° ≤view< 45° or a view angular range containing even a periphery with this view as a principal part, and 135° ≤view< 225° or a view angular range containing even a periphery with this view as a principal part, and a yz plane passing through the center of rotation is configured as the projection plane in 45° ≤view< 135° or a view angular range containing even a periphery with this view as a principal part, and 225° ≤view< 315° or a view angular range containing even a periphery with this view as a principal part.

According to a seventh aspect, the present invention provides an image reconstructing method wherein in the image reconstructing method having the above configuration, one plane-projected data D1 is determined from the plurality of projection data D0 by an interpolation/extrapolation process.

According to an eighth aspect, the present invention provides an image reconstructing method wherein in the image reconstructing method having the above configuration, addresses and interpolation/extrapolation factors for the plurality of projection data D0 for determining one plane-projected data D1 are tabulated.

According to a ninth aspect, the present invention provides an image reconstructing method wherein in the image reconstructing method having the above configuration, one plane-projected data D1 is determined from the plurality of projection data D0 by an interpolation/extrapolation process, addresses and interpolation/extrapolation factors for the plurality of projection data D0 for determining one plane-projected data D1 are arranged in table form in any one of -45° ≤view< 45° or a view angular range containing even a periphery with this view as a principal part, 135° ≤view< 225° or a view angular range containing even a periphery with this view as a principal part, 45° ≤view< 135° or a view angular range containing even a periphery with this view as a principal part, and 225° ≤view< 315° or a view angular range containing even a periphery with this view as a principal part, and the table is utilized in other view angular ranges.

According to a tenth aspect, the present invention provides an image reconstructing method wherein in the image reconstructing method having the above configuration, the interpolation/extrapolation process includes a 0-order interpolation/extrapolation process or a primary interpolation/extrapolation process.

According to an eleventh aspect, the present invention provides an image reconstructing method wherein in the image reconstructing method having the above configuration, one backprojected pixel data D2 is determined by a weight adding process of the plurality of plane-projected data D1.

According to a twelfth aspect, the present invention provides an image reconstructing method wherein in the image reconstructing method having the above configuration, the weight of the weight adding process is determined according to a distance from an X-ray focal point to each plane-projected data D1.

According to a thirteenth aspect, the present invention provides an image reconstructing method wherein in the image reconstructing method having the above configuration, the weight of the weight adding process is determined according to a distance from the X-ray focal point to each of the pixels in the reconstruction area.

According to a fourteenth aspect, the present invention provides an image reconstructing method wherein in the image reconstructing method having the above configuration, the weight of the weight adding process is common to the respective pixels lying in the reconstruction area and the pixels lying on the straight lines parallel to the projection plane.

According to a fifteenth aspect, the present invention provides an image reconstructing method wherein in the image reconstructing method having the above configuration, a start address, a sampling pitch and the number of samplings are determined and the plane-projected data D1 are sampled to select the plane-projected data D1 for effecting the weight adding process on the respective pixels lying in the reconstruction area and the pixels lying on the straight lines parallel to the projection plane.

According to a sixteenth aspect, the present invention provides an image reconstructing method wherein in the image reconstructing method having the above configuration, the weight of the weight adding process, the start address, the sampling pitch and the number of the samplings are determined in advance and tabulated.

According to a seventeenth aspect, the present invention provides an image reconstructing method wherein in the image reconstructing method having the above configuration, a result obtained by multiplying backprojected pixel data D2 of a given view and backprojected pixel data D2 of an opposite view by weighting factors ωa and ωb (where ωa + ωb = 1) corresponding to angles formed by the straight lines connecting the respective pixels of the reconstruction area at both views and the X-ray focal point and the plane containing the reconstruction area and by adding the same together is set as backprojected pixel data D2 of a given view.

According to an eighteenth aspect, the present invention provides an X-ray CT apparatus comprising an X-ray tube; a multi-row detector opposite to the X-ray tube and having a plurality of detector sequences; linear movement control means for relatively moving the X-ray tube and the multi-row detector linearly with a subject along a linear travel axis; rotation control means for rotating at least one of the X-ray tube and the multi-row detector about a rotational axis; tilt control means for tilting an angle of a scan surface formed by the rotation with respect to the linear travel axis to an angular slope other than 90°; scan control means for collecting projection data D0, using the multi-row detector and by a helical scan with a scan surface being tilted; and image reconstructing means for reconstructing an image, based on the projection data D0, wherein the image reconstructing means includes tilt correcting means for effecting a tilt correcting process for correcting variations every views, in positions of respective channels of the detector sequences with respect to the linear travel axis due to the inclination of the scan surface, on the projection data D0, backprojected pixel data calculating means for then projecting respective pixels in an X-ray penetration direction along lines parallel to an X axis or a Y axis on a reconstruction plane (XY plane) to determine the corresponding projection data D0 and thereby determining backprojected pixel data D2 of respective pixels constituting the reconstruction plane; and backprojected data calculating means for adding the backprojected pixel data D2 of all views used in image reconstruction in association with the pixels to determine backprojected data D3.

According to a nineteenth aspect, the present invention provides an X-ray CT apparatus further comprising fan-para converting means for determining projection data D0p corresponding to parallel data from projection data D0f corresponding to fan data, wherein in the X-ray CT apparatus having the above configuration, the scan control means collects the projection data D0f corresponding to the fan data, and the tilt correcting means effects a tilt correcting process on the projection data D0p corresponding to the parallel data.

According to a twentieth aspect, the present invention provides an X-ray CT apparatus comprising an X-ray tube; a multi-row detector opposite to the X-ray tube and having a plurality of detector sequences; linear movement control means for relatively moving the X-ray tube and the multi-row detector linearly with a subject along a linear travel axis; rotation control means for rotating at least one of the X-ray tube and the multi-row detector about a rotational axis; tilt control means for tilting an angle of a scan surface formed by the rotation with respect to the linear travel axis to an angular slope other than 90°; scan control means for collecting projection data D0, using the multi-row detector and by a helical scan with a scan surface being tilted; and image reconstructing means for reconstructing an image, based on the projection data D0, wherein the image reconstructing means includes tilt correcting means for effecting a tilt correcting process for correcting variations every views, in positions of respective channels of the detector sequences with respect to the linear travel axis due to the inclination of the scan surface, on the projection data D0, plane-projected data calculating means for determining data D1 plane-projected onto the plane of projection on the basis of the tilt-corrected projection data D0, backprojected pixel data calculating means for projecting, in an X-ray penetration direction, the plane-projected data D1 onto respective pixels constituting a plurality of lines which are placed on a reconstruction area with plural pixel intervals defined thereamong and extend in the direction parallel to the projection plane to thereby determine backprojected pixel data D2 of the respective pixels constituting the lines on the reconstruction area, and interpolating among the plurality of lines to determine backprojected pixel data D2 of respective pixels among the lines on the reconstruction area, and backprojected data calculating means for adding the backprojected pixel data D2 of all views employed in image reconstruction in association with the pixels to determine backprojected data D3.

According to a twenty-first aspect, the present invention provides an X-ray CT apparatus comprising an X-ray tube; a multi-row detector opposite to the X-ray tube and having a plurality of detector sequences; linear movement control means for relatively moving the X-ray tube and the multi-row detector linearly with a subject along a linear travel axis; rotation control means for rotating at least one of the X-ray tube and the multi-row detector about a rotational axis; tilt control means for tilting an angle of a scan surface formed by the rotation with respect to the linear travel axis to an angular slope other than 90°; scan control means for collecting projection data D0, using the multi-row detector and by a helical scan with a scan surface being tilted; and image reconstructing means for reconstructing an image, based on the projection data D0, wherein the image reconstructing means includes tilt correcting means for effecting a tilt correcting process for correcting variations every views, in positions of respective channels of the detector sequences with respect to the linear travel axis due to the inclination of the scan surface, on the projection data D0, plane-projected data calculating means for determining data D1 plane-projected onto lines on a projection plane, corresponding to a plurality of lines which are placed on a reconstruction area with plural pixel intervals defined thereamong and extend in the direction parallel to the projection plane, on the basis of the tilt-corrected projection data D0, backprojected pixel data calculating means for determining backprojected pixel data D2 of respective pixels on the reconstruction area on the basis of the plane-projected data D1, and backprojected data calculating means for adding the backprojected pixel data D2 of all views used in image reconstruction in association with the pixels to determine backprojected data D3.

According to a twenty-second aspect, the present invention provides an X-ray CT apparatus wherein in the X-ray CT apparatus having the above configuration, the number of the lines ranges from 1/64 to 1/2 of the number of the pixels in the reconstruction area as viewed in the direction orthogonal to the lines.

According to a twenty-third aspect, the present invention provides an X-ray CT apparatus wherein in the X-ray CT apparatus having the above configuration, when the direction orthogonal to a rotating plane of the X-ray tube or the multi-row detector is defined as a z direction, the direction of a center axis of an X-ray beam at view = 0° is defined as a y direction, and the direction orthogonal to the z direction and the y direction is defined as an x direction, the plane-projected data calculating means sets an xz plane passing through the center of rotation as the projection plane in -45° ≤view< 45° or a view angular range containing even a periphery with this view as a principal part, and 135° ≤view< 225° or a view angular range containing even a periphery with this view as a principal part, and sets a yz plane passing through the center of rotation as the projection plane in 45° ≤view< 135° or a view angular range containing even a periphery with this view as a principal part, and 225° ≤view< 315° or a view angular range containing even a periphery with this view as a principal part.

According to a twenty-fourth aspect, the present invention provides an X-ray CT apparatus wherein in the X-ray CT apparatus having the above configuration, the plane-projected data calculating means determines one plane-projected data D1 from the plurality of projection data D0 by an interpolation/extrapolation process.

According to a twenty-fifth aspect, the present invention provides an X-ray CT apparatus wherein in the X-ray CT apparatus having the above configuration, the plane-projected data calculating means uses a table to which addresses and interpolation/extrapolation factors for the plurality of projection data D0 for determining one plane-projected data D1 are set.

According to a twenty-sixth aspect, the present invention provides an X-ray CT apparatus wherein in the X-ray CT apparatus having the above configuration, the plane-projected data calculating means determines one plane-projected data D1 from the plurality of projection data D0 by an interpolation/extrapolation process, tabulates addresses and interpolation/extrapolation factors for the plurality of projection data D0 for determining one plane-projected data D1 in any one of -45° ≤view< 45° or a view angular range containing even a periphery with this view as a principal part, 135° ≤view< 225° or a view angular range containing even a periphery with this view as a principal part, 45° ≤view< 135° or a view angular range containing even a periphery with this view as a principal part, and 225° ≤view< 315° or a view angular range containing even a periphery with this view as a principal part, and makes use of the resultant table in other view angular ranges.

According to a twenty-seventh aspect, the present invention provides an X-ray CT apparatus wherein in the X-ray CT apparatus having the above configuration, the interpolation/extrapolation process includes a 0-order interpolation/extrapolation process or a primary interpolation/extrapolation process.

According to a twenty-eighth aspect, the present invention provides an X-ray CT apparatus wherein in the X-ray CT apparatus having the above configuration, one backprojected pixel data D2 is determined by a weight adding process of the plurality of plane-projected data D1.

According to a twenty-ninth aspect, the present invention provides an X-ray CT apparatus wherein in the X-ray CT apparatus having the above configuration, the weight of the weight adding process is determined according to a distance from each of the pixels in the reconstruction area to each plane-projected data D1.

According to a thirtieth aspect, the present invention provides an X-ray CT apparatus wherein in the X-ray CT apparatus having the above configuration, the weight of the weight adding process is determined according to a distance from each of the pixels in the reconstruction area to the X-ray focal point.

According to a thirty-first aspect, the present invention provides an X-ray CT apparatus wherein in the X-ray CT apparatus having the above configuration, the weight of the weight adding process is common to the respective pixels lying in the reconstruction area and the pixels lying on the straight lines parallel to the projection plane.

According to a thirty-second aspect, the present invention provides an X-ray CT apparatus wherein in the X-ray CT apparatus having the above configuration, a start address, a sampling pitch and the number of samplings are determined, and the plane-projected data D1 are sampled to continuously select the plane-projected data D1 for effecting the weight adding process on the respective pixels lying in the reconstruction area and the pixels lying on the straight lines parallel to the projection plane.

According to a thirty-third aspect, the present invention provides an X-ray CT apparatus wherein in the X-ray CT apparatus having the above configuration, the weight of the weight adding process, the start address, the sampling pitch and the number of the samplings are determined in advance and tabulated.

According to a thirty-fourth aspect, the present invention provides an X-ray CT apparatus wherein in the X-ray CT apparatus having the above configuration, the weight of the weight adding process is determined according to an angle formed by a straight line connecting each pixel of a reconstruction area at a given view and an X-ray focal point and a plane containing the reconstruction area, and an angle formed by a straight line connecting each pixel of the reconstruction area at an opposite view and an X-ray focal point and a plane containing the reconstruction area.

In the image reconstructing method according to the first aspect, a tilt correcting process for correcting variations every views, in positions of respective channels of detector sequences with respect to a linear travel axis due to the inclination of a scan surface is effected on the projection data D0. Then, respective pixels are projected in an X-ray penetration direction along lines parallel to an X axis or a Y axis on a reconstruction plane (XY plane) to determine the corresponding projection data D0, thereby determining backprojected pixel data D2 of respective pixels constituting the reconstruction plane. Thus, reconstruction can be performed at high speed using projection data properly corresponding to an X-ray beam transmitted through a reconstruction area.

In the image reconstructing method according to the second aspect, projection data D0p corresponding to parallel data are determined from projection data D0f corresponding to fan data without directly determining plane-projected data D1 from the projection data D0f corresponding to the fan data, and the plane-projected data D1 are determined from the projection data D0p corresponding to the parallel data.

When the plane-projected data D1 are determined directly from the projection data D0f corresponding to the fan data here, it was necessary to take into consideration the distance from an X-ray focal point to a channel corresponding to each projection data D0f and the distance from the X-ray focal point to a projection position on a projection plane. That is, there was a need to multiply the data by distance factors. Since, however, there is no need to perform multiplication of the distance factors where the plane-projected data D1 are determined from the projection data D0p corresponding to the parallel data, computing can be simplified. It was not possible to make a contrivance to handle the opposite view in the case of the projection data D0f corresponding to the fan data. However, the projection data D0p corresponding to the parallel data make it easy to handle the opposite view. Therefore, the utilization of the opposite view shifted by a -1/4 channel and the original view shifted by a+1/4 channel in combination makes it possible to enhance resolution in a channel direction, reduce the number of views at backprojection to 1/2 and lessen a calculated amount.

In the image reconstructing method according to the third aspect, plane-projected data D1 are determined from tilt-corrected projection data D0, and the plane-projected data D1 are projected onto a reconstruction area in an X-ray penetration direction to determine backprojected pixel data D2. Thus, reconstruction can be done at high speed using projection data properly associated with an X-ray beam transmitted through the reconstruction area.

Incidentally, while the reconstruction area is of a plane, a multi-row detector is located in an arcuate spatial position. When data located in the arcuate form are directly projected onto a reconstruction area corresponding to lattice coordinates, a coordinate transformation process becomes complicated and hence a calculated amount is needed. Further, when this processing is done over all pixels of the reconstruction area, an enormous amount of calculation is required. That is, the direct determination of the projection data D0 from the backprojected pixel data D2 makes processing complicated and also lengthens a processing time interval.

In contrast, in the image reconstructing method according to the third aspect, plane-projected data D1 are determined from projection data D0 without directly determining backprojected pixel data D2 from the projection data D0, and the backprojected pixel data D2 are determined from the plane-projected data D1. When data located in the plane is projected onto a reconstruction area corresponding to lattice coordinates here, the processing is done by primary or linear transformation (affine transformation) capable of realizing the processing by data sampling with an equi-sampling pitch. Thus, the simplification and speeding-up of the processing are enabled looking overall. Incidentally, the plane-projected data D1 may preferably be set to intervals sufficiently close in a channel direction of at least a detector.

Further, when backprojected pixel data D2 are determined from plane-projected data D1, only backprojected pixel data D2 on respective pixels constituting lines which are placed on a reconstruction area with plural pixel intervals defined thereamong and extend in the direction parallel to the plane of projection are determined, and an interpolation process is applied among the lines arranged at plural pixel intervals. Therefore, a processing time interval can be shortened as compared with the case in which the backprojected pixel data D2 on all the pixels constituting the reconstruction area are determined from the plane-projected data D1. Incidentally, if the number of lines at the plural pixel intervals is properly selected, then deterioration in image quality can be suppressed to a negligible degree.

In the image reconstructing method according to the fourth aspect, plane-projected data D1 are determined from tilt-corrected projection data D0, and the plane-projected data D1 are projected on a reconstruction area in an X-ray penetration direction to determine backprojected pixel data D2. Thus, reconstruction can be carried out at high speed using projection data properly corresponding to an X-ray beam transmitted through the reconstruction area.

Incidentally, while the reconstruction area is of a plane, a multi-row detector is located in an arcuate spatial position. When data located in the arcuate form are directly projected on a reconstruction area corresponding to lattice coordinates, a coordinate transformation process becomes complicated and hence a calculated amount is needed. Further, when this processing is done over all pixels of the reconstruction area, an enormous amount of calculation is required. That is, the direct determination of the projection data D0 from the backprojected pixel data D2 makes processing complicated and also lengthens a processing time interval.

In contrast, in the image reconstructing method according to the fourth aspect, plane-projected data D1 are determined from projection data D0 without directly determining backprojected pixel data D2 from the projection data D0, and the backprojected pixel data D2 are determined from the plane-projected data D1. When data located in the plane is projected onto a reconstruction area corresponding to lattice coordinates here, the processing is done by primary or linear transformation (affine transformation) capable of realizing the processing by data sampling with an equi-sampling pitch. Thus, the simplification and speeding-up of the processing are enabled looking overall. Incidentally, the plane-projected data D1 may preferably be set to intervals sufficiently close in a channel direction of at least a detector.

Further, when the plane-projected data D1 are determined, unnecessary computing can be omitted upon determining data D1 plane-projected on lines on the plane of projection, corresponding to a plurality of lines which are placed on a reconstruction area with plural pixel intervals defined thereamong and extend in the direction parallel to the plane of projection. Therefore, a processing time interval can be shortened. Incidentally, if the number of lines placed at the plural pixel intervals is selected properly, then deterioration in the image quality can be suppressed to a negligible degree.

In the image reconstructing method according to the fifth aspect, the number of the lines at the plural pixel intervals is set so as to range from 1/64 to 1/2 of the number of pixels of a reconstruction area as viewed in the direction normal to each line, so that the shortening effect of processing time and deterioration in image quality can be suitably kept in balance.

In the image reconstructing method according to the sixth aspect, the angle which an xz plane or a yz plane corresponding to the plane of projection forms with a projection-directed line is not smaller than about 45°. Therefore, a reduction in the accuracy of calculation can be suppressed to within an allowable range.

Incidentally, view = -45° and view = 315° are actually equal and the same view although they are described in different expressions for convenience of expression in the present specification. When data are projected onto the plane of projection, the accuracy becomes high as the angle formed by its projection-directed line and the plane of projection approaches 90°, whereas the accuracy becomes low as the angle approaches 0°.

In the image reconstructing method according to the seventh aspect, one plane-projected data D1 is determined from a plurality of projection data D0 by an interpolation process. Therefore, the density of the plane-projected data D1 can be made high sufficiently as compared with a pixel density of a reconstruction area. Thus, the process of projecting the plane-projected data D1 onto the corresponding reconstruction area as seen in an X-ray penetration direction to determine backprojected pixel data D2 is intended for the most proximity affine transformation process, i. e., sampling process alone, and hence the interpolation process can be eliminated, thereby making it possible to achieve simplification and speeding up of processing. However, the interpolation process may be done if desired.

In the image reconstructing method according to the eighth aspect, addresses and interpolation/extrapolation factors for a plurality of projection data D0 are calculated in advance and set to a table, so that the overhead can be eliminated. That is, processing can be speeded up by tabulation. Incidentally, the addresses and interpolation/extrapolation factors for the plurality of projection data D0 for determining one plane-projected data D1 may be calculated every attempt to determine one plane-projected data D1. However, the time required for its calculation results in overhead.

When a geometrical relationship among an X-ray tube, a detector and a projection axis in 135° ≤view< 225° or a view angular range containing even a periphery with this view as a principal part is rotated by 180° about the center of rotation where an xz plane passing through the center of rotation is configured as the plane of projection, it coincides with a geometrical relationship among the X-ray tube, the detector and the projection axis in -45° ≤view< 45° or a view angular range containing even a periphery with this view as a principal part. Thus, addresses and interpolation/extrapolation factors for projection data D0 for determining one plane-projected data D1 can be shared between the two.

When a geometrical relationship among the X-ray tube, the detector and the projection axis in 45° ≤view< 135° or a view angular range containing even a periphery with this view as a principal part is rotated by -90° about the center of rotation where a yz plane passing through the center of rotation is configured as the plane of projection, it coincides with a geometrical relationship among the X-ray tube, the detector and the projection axis in -45° ≤view< 45° or a view angular range containing even a periphery with this view as a principal part where the xz plane passing through the center of rotation is set as the plane of projection. Thus, addresses and interpolation/extrapolation factors for projection data D0 for determining one plane-projected data D1 can be shared between the two.

When a geometrical relationship among the X-ray tube, detector and projection axis in 225° ≤view< 315° or a view angular range containing even a periphery with this view as a principal part is rotated by 90° about the center of rotation where the yz plane passing through the center of rotation is configured as the plane of projection, it coincides with a geometrical relationship among the X-ray tube, the detector and the projection axis in -45° ≤view< 45° or a view angular range containing even a periphery with this view as a principal part where the xz plane passing through the center of rotation is set as the plane of projection. Thus, addresses and interpolation/extrapolation factors for projection data D0 for determining one plane-projected data D1 can be shared between the two.

In the image reconstructing method according to the ninth aspect, a table used in any one of -45° ≤view< 45° or a view angular range containing even a periphery with this view as a principal part, 135° ≤view< 225° or a view angular range containing even a periphery with this view as a principal part, 45° ≤view< 135° or a view angular range containing even a periphery with this view as a principal part, and 225° ≤view< 315° or a view angular range containing even a periphery with this view as a principal part can be shared even in other view angular ranges. It is therefore possible to reduce memory capacity necessary for the table.

In the image reconstructing method according to the tenth aspect, an interpolation/extrapolation process can be simply handled because a 0-order interpolation/extrapolation process (i.e., adoption of proximity data) and a primary interpolation/extrapolation process (i.e., interpolation/extrapolation using two proximity data) are included.

In the image reconstructing method according to the eleventh aspect, the weight addition of plural data of the same view or opposite view near a reconstruction area is applicable. In the image reconstructing method according to the twelfth aspect, backprojected pixel data D2 can be properly determined. This is because data D1 in which the distance from an X-ray focal point to plane-projected data D1 is short, are generally considered to include information about respective pixels more properly as compared with data D1 long in distance.

In the image reconstructing method according to the thirteenth aspect, backprojected pixel data D2 can be determined more properly. This is because the distance from an X-ray focal point to a detector is constant and hence data D1 at the time that the distance from each of pixels of a reconstruction area to the X-ray focal point is long, are considered to be near the detector in distance and to include information about respective pixels more properly as compared with data D1 at the time that the distance is short.

In the image reconstructing method according to the fourteenth aspect, the weight is used in common and processing can be simplified. The weight of a weight adding process can be defined as the ratio between the distance from an X-ray focal point to plane-projected data D1 and the distance from the X-ray focal point to each of pixels of a reconstruction area. In this case, the ratio becomes identical in value between each of the pixels of the reconstruction area and each of pixels located on straight lines parallel to the plane of projection.

In the image reconstructing method according to the fifteenth aspect, plane-projected data D1 for determining backprojected pixel data D2 can be selected by a simple process. This is because plane-projected data D1 for determining backprojected pixel data D2 about respective pixels of a reconstruction area and pixels located on straight lines parallel to the plane of projection exist on the lines on the plane of projection. Thus, if a start address, a sampling pitch and the number of samplings are fixed, then the data can be selected by simple processing.

In the image reconstructing method according to the sixteenth aspect, processing can be speeded up by tabulation. In the image reconstructing method according to the seventeenth aspect, backprojected pixel data D2 can be determined more properly. This is because information about respective pixels are generally considered to be contained more properly as the angle which a straight line connecting each pixel of a reconstruction area and an X-ray focal point forms with a plane containing the reconstruction area, approaches 90°.

The X-ray CT apparatus according to the eighteenth aspect is capable of suitably implementing the image reconstructing method according to the first aspect. The X-ray CT apparatus according to the nineteenth aspect is capable of suitably implementing the image reconstructing method according to the second aspect. The X-ray CT apparatus according to the twentieth aspect is capable of suitably implementing the image reconstructing method according to the third aspect. The X-ray CT apparatus according to the twenty-first aspect is capable of suitably implementing the image reconstructing method according to the fourth aspect. The X-ray CT apparatus according to the twenty-second aspect is capable of suitably implementing the image reconstructing method according to the fifth aspect. The X-ray CT apparatus according to the twenty-third aspect is capable of suitably implementing the image reconstructing method according to the sixth aspect. The X-ray CT apparatus according to the twenty-fourth aspect is capable of suitably implementing the image reconstructing method according to the seventh aspect.

The X-ray CT apparatus according to the twenty-fifth aspect is capable of suitably implementing the image reconstructing method according to the eighth aspect. The X-ray CT apparatus according to the twenty-sixth aspect is capable of suitably implementing the image reconstructing method according to the ninth aspect. The X-ray CT apparatus according to the twenty-seventh aspect is capable of suitably implementing the image reconstructing method according to the tenth aspect. The X-ray CT apparatus according to the twenty-eighth aspect is capable of suitably implementing the image reconstructing method according to the eleventh aspect. The X-ray CT apparatus according to the twenty-ninth aspect is capable of suitably implementing the image reconstructing method according to the twelfth aspect.

The X-ray CT apparatus according to the thirtieth aspect is capable of suitably implementing the image reconstructing method according to the thirteenth aspect. The X-ray CT apparatus according to the thirty-first aspect is capable of suitably implementing the image reconstructing method according to the fourteenth aspect. The X-ray CT apparatus according to the thirty-second aspect is capable of suitably implementing the image reconstructing method according to the fifteenth aspect. The X-ray CT apparatus according to the thirty-third aspect is capable of suitably implementing the image reconstructing method according to the sixteenth aspect. The X-ray CT apparatus according to the thirty-fourth aspect is capable of suitably implementing the image reconstructing method according to the seventeenth aspect.

Further objects and advantages of the present invention will be apparent from the following description of the preferred embodiments of the invention as illustrated in the accompanying drawings, in which:

Fig. 1 is a block diagram of an X-ray CT apparatus.

Fig. 2 is a flowchart of an image reconstructing process.

Fig. 3 is a flowchart of a tilt correcting process.

Fig. 4 is an explanatory diagram showing positions relative to a linear travel axis of an ith channel at a view angle $\phi = 0$.

Fig. 5 is an explanatory diagram showing positions relative to a linear travel axis of an ith channel at a view angle $\phi = \pi/2$.

Fig. 6 is an explanatory diagram illustrating the manner in which the positions relative to the linear travel axis of the ith channel vary every views due to the inclination of a scan surface.

Fig. 7 is an explanatory diagram showing a jth detector sequence at a view angle $\phi = \pi/2$.

Fig. 8 is an explanatory diagram of a data position moving process on a first detector sequence of a multi-row detector.

Fig. 9 is an explanatory diagram of a data position moving process on a second detector sequence of the multi-row detector.

Fig. 10 is an explanatory diagram of a data cutting-out process on the first detector sequence of the multi-row detector.

Fig. 11 is an explanatory diagram of a dummy data adding process on the first detector sequence of the multi-row detector.

Fig. 12 is an explanatory diagram of a data cutting-out process on the second detector sequence of the multi-row detector.

Fig. 13 is an explanatory diagram of a dummy data adding process on the second detector sequence of the multi-row detector.

Fig. 14 is an explanatory diagram of a data converting process by a linear interpolating computation.

Fig. 15 is a flowchart of another example of the tilt correcting process.

Fig. 16 is a flowchart of a three-dimensional back projecting process.

Fig. 17 is an explanatory diagram showing the layout of an X-ray tube and a multi-row detector at view = 0° and $\delta$ = 0° and plane-projected data.

Fig. 18 is an explanatory diagram showing the layout of the X-ray tube and multi-row detector at view = 0° and $\delta$ = 360° and plane-projected data.

Fig. 19 is an explanatory diagram showing plane-projected data at view = 0°.

Fig. 20 is an explanatory diagram showing plane-projected data at view = 0° subsequent to an interpolation/extrapolation process in a qt direction.

Fig. 21 is an explanatory diagram showing plane-projected data at view = 30°.

Fig. 22 is an explanatory diagram showing plane-projected data at view = 30° subsequent to the interpolation/extrapolation process in the qt direction.

Fig. 23 is an explanatory diagram showing the layout of the X-ray tube and multi-row detector at view = 90° and plane-projected data.

Fig. 24 is a diagram illustrating a lookup table for calculation of plane-projected data.

Fig. 25 is an explanatory diagram showing repetitive units for the interpolation/extrapolation process in the qt direction.

Fig. 26 is a diagram illustrating a spatial position of a reconstruction area.

Fig. 27 is an explanatory diagram showing a state in which plane-projected data at view = 0° is projected onto the reconstruction area in an X-ray penetration direction to determine backprojected pixel data.

Fig. 28 is a diagram illustrating a lookup table for back projection.

Fig. 29 is an explanatory diagram showing a case in which backprojected pixel data about pixels on a line on the reconstruction area and parallel to the plane of projection are determined.

Fig. 30(a) is a conceptual diagram showing backprojected pixel data D2 about pixels on a plurality of lines with plural pixel intervals defined thereamong on a reconstruction area at view = 0° and parallel to the plane of projection, and Fig. 30(b) is a conceptual diagram showing backprojected pixel data D2 of an opposite view.

Fig. 31(a) is a conceptual diagram showing backprojected pixel data D2 about pixels on a plurality of lines with plural pixel intervals defined thereamong on a reconstruction area at view = 0° and parallel to the plane of projection, and Fig. 31 (b) is an explanatory diagram of backprojected pixel data D2 about all pixels in the reconstruction area at view = 0° which are obtained by interpolating among the lines.

Fig. 32 is an explanatory diagram showing a state in which the backprojected pixel data D2 are added corresponding to pixels over all views to obtain backprojected data D3.

Fig. 33(a) is a conceptual diagram showing a plurality of lines with plural pixel intervals defined thereamong on a reconstruction area at view = 0° and parallel to the plane of projection, and Fig. 33(b) is a conceptual diagram showing lines on the plane of projection corresponding to the plurality of lines with the plural pixel intervals defined thereamong on the reconstruction area at view = 0° and parallel to the plane of projection.

Fig. 34 is a conceptual diagram showing lines on the plane of projection corresponding to a plurality of lines with plural pixel intervals defined thereamong on a reconstruction area at view = 0° and parallel to the plane of projection.

Fig. 35 is a conceptual diagram showing a process for determining plane-projected data D1 about lines on the plane of projection corresponding to a plurality of lines with plural pixel intervals defined thereamong on a reconstruction area at view = 0° and parallel to the plane of projection.

Fig. 36 is a diagram illustrating a lookup table for back projection.

Fig. 37 is a conceptual diagram showing a process for sampling plane-projected data D1 about lines on the plane of projection to determine backprojected pixel data D2 about the lines on a reconstruction area.

Fig. 38 is a conceptual diagram showing a process for effecting an interpolation process on plane-projected data D1 about lines on the plane of projection to determine backprojected pixel data D2 about the lines on a reconstruction area.

Fig. 39 is a schematic flowchart for describing the operation of an X-ray CT apparatus.

Fig. 40 is a flowchart for describing a fan-para converting process.

Fig. 41 is a sinogram showing the conception of the fan-para converting process.

Fig. 42 is a conceptual diagram showing X-ray penetration paths and channels corresponding to parallel data.

Fig. 43 is a conceptual diagram showing opposite views of the parallel data.

Fig. 44 is a conceptual diagram showing parallel data in which densities in a channel direction are equalized.

Fig. 45 is a flowchart showing another example of the three-dimensional back projecting process.

Fig. 46 is an explanatory diagram showing a state in which projected data D0p of fan data at view = 0° is projected onto the plane of projection in an X-ray penetration direction to determine plane-projected data D1.

Fig. 47 is an explanatory diagram showing a state in which plane-projected data D1 at view = 0° is projected onto a reconstruction area P in an X-ray penetration direction to determine backprojected pixel data D2.

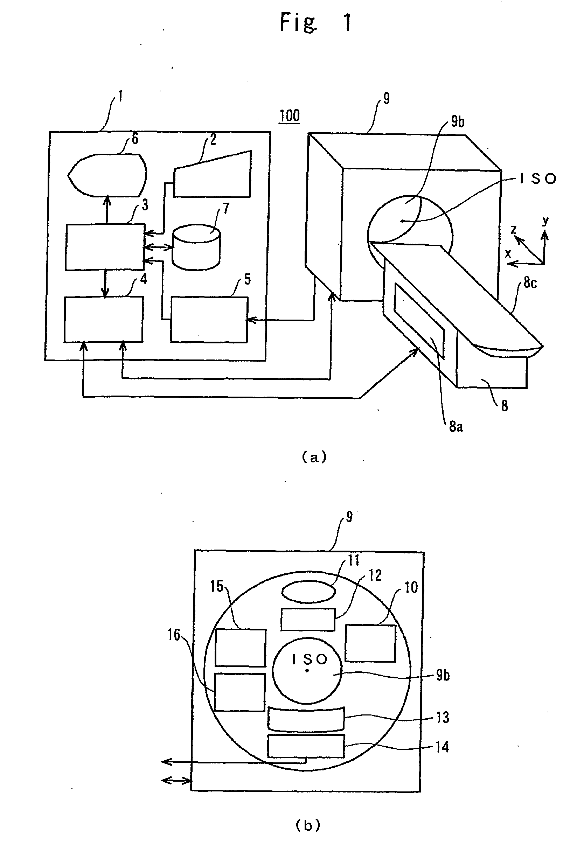

Best modes for carrying out the invention will be explained below with reference to the accompanying drawings. Incidentally, the present invention is not limited to the best modes for carrying out the invention. A block diagram of an X-ray CT apparatus is shown in Fig. 1. The present apparatus is one example of the best mode for carrying out the present invention. One example of the best mode for carrying out the present invention related to the X-ray CT apparatus is shown by the configuration of the present apparatus. One example of the best mode for carrying out the present invention related to an image reconstructing method is shown by the operation of the present apparatus.

As shown in Fig. 1, the X-ray CT apparatus 100 is equipped with an operating console 1, a table device 8 and a scanning gantry 9. The operating console 1 is provided with an input device 2 which receives an instruction input and an information input or the like from an operator, a central processing unit 3 which executes a scan process, an image reconstructing process, etc., a control interface 4 which performs a transfer of a control signal or the like between the imaging table 8 and the scanning gantry 9, a data acquisition buffer 5 which collects data obtained by the scanning gantry 9, a CRT 6 which displays an image reconstructed from the data, and a memory device 7 which stores programs, data and an image therein.

The table device 8 includes a cradle 8c which places a subject thereon and a transfer controller 8a for moving the cradle 8c in a z-axis direction and a y-axis direction. Incidentally, a y axis is defined as a vertical direction and a z axis is defined as the longitudinal direction of the cradle 8c. An axis orthogonal to the y axis and the z axis is defined as an x axis. A body axis of the subject is placed in the z-axis direction.

The scanning gantry 9 is provided with an X-ray controller 10, an X-ray tube 11, a collimator 12, a multi-row detector 13 having a plurality of detector sequences, a data acquisition unit 14, a rotation controller 15 for rotating the X-ray tube 11 and the multi-row detector 13 or the like about an isocentre ISO, and a tilt controller 16 for making the slope of an angle of a scan surface. The tilt controller 16 controls the slope of the scanning gantry 9.

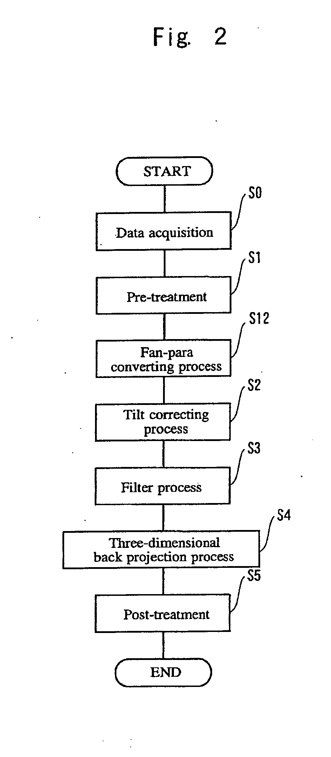

Fig. 2 is a flowchart showing a schematic flow of the operation of the X-ray CT apparatus 100. In Step S0, projection data D0(view, $\delta$, j, i) represented by a view angle View, a relative angle difference $\delta$, a detector sequence number j and a channel number i are collected while the X-ray tube 21 and the multi-row detector 24 are being rotated about a subject or object to be imaged in a state they are being slanted, and the cradle 12 is being moved linearly. The collected projection data are converted to parallel data by fan-para conversion. Incidentally, the relative angle difference $\delta$ is a parameter indicating to what number the rotation corresponds at the same view. For instance, a second rotation is expressed in $\delta = 360°$.

In Step S 1, a pre-treatment (offset correction, logarithmic correction, X-ray dose correction and sensitivity correction) is effected on the projection data D0(view, $\delta$, j, i). In Step S2, a tilt correcting process is performed on the projection data D0(view, $\delta$, j, i). The tilt correcting process will be explained again later.

In Step S3, a filter process is effected on the projection data D0(view, $\delta$, j, i) subjected to the tilt correcting process. That is, a Fourier transform is performed on the projection data, each of which is followed by being multiplied by a filter (reconstruction function) to perform an inverse Fourier transform thereof. In Step S4, a three-dimensional back projecting process according to the present invention is effected on the projection data D0(view, $\delta$, j, i) subjected to the filter process to determine backprojected data D3(x, y). The three-dimensional back projecting process will be explained again later. In Step S5, a post-treatment is effected on the backprojected data D3(x, y) to obtain a CT image.

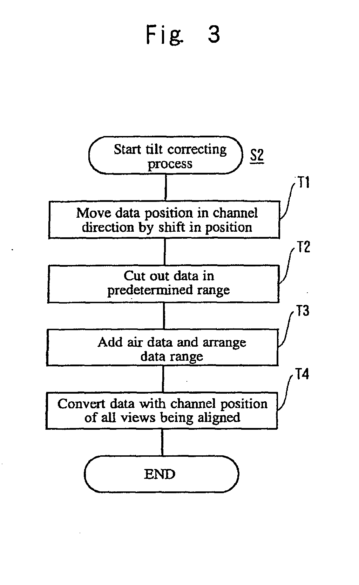

The tilt correcting process will be explained. Fig. 3 is a flowchart showing the tilt correcting process (S2). In Step T1, data are arranged in the form of a two-dimension of a channel number axis and a view number axis. Next, the positions of the data are moved so as to counteract the effect that the positions of respective channels of detector sequences with respect to the linear travel axis vary every views due to the slope of the scan surface. A specific example of the data position moving process will be explained later.

In Step T2, data lying in a range in which data exist over all views as seen in a view direction under a post-movement data array, are cut out. A specific example of the data cutting-out process will be explained later. In Step T3, dummy data is added to the cut-out data to fix up a data range. A specific example of the dummy data adding process will be explained later. In Step T4, the data are converted to data in which channel positions of all views are aligned with one another. A specific example of the data converting process will be explained later.

The specific examples will next be described. Now consider that a helical scan is rotated by substantially $2\pi$

over all views (corresponding to one cycle) and linearly moved by a slice width under the rotation of $2\pi$ (helical pitch = 1), a tilt angle is defined $\theta$ and the distance from an intersecting point (isocenter ISO) of a linear travel axis and a rotational axis to a scan surface corresponding to a jth detector sequence is defined as Lj. A view angle at the time that the multi-row detector 13 is located directly below is defined as $\phi$ = 0, and a view number is defined as pvn = 1.

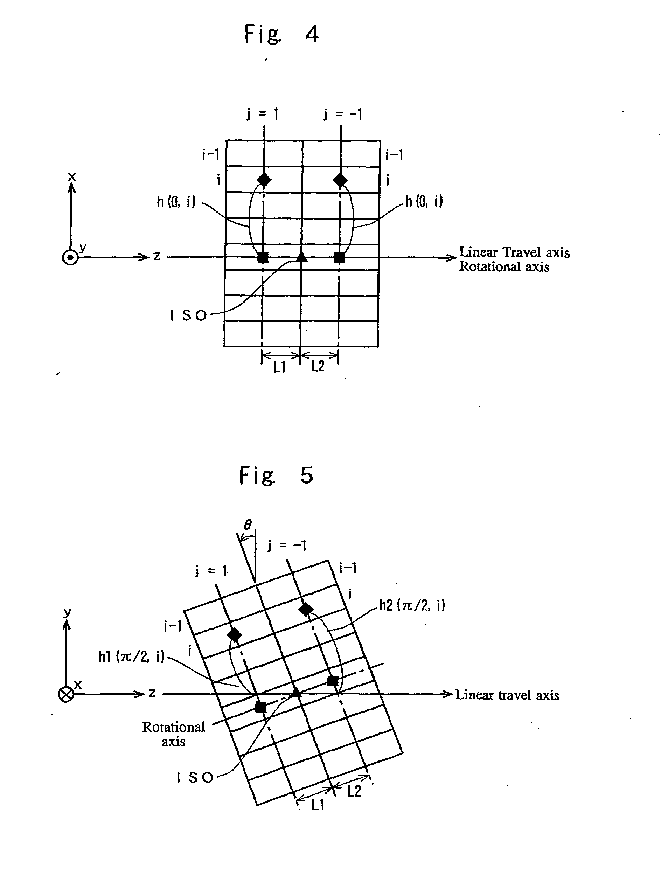

Fig. 4 shows the multi-row detector at the view angle $\phi$ = 0. However, the number of detector sequences is expressed in two rows. A position h(0, i) of an ith channel of a first detector sequence (j = 1) relative to a linear travel axis and a position h(0, i) of an ith channel of a second detector sequence (j = -1) relative to the linear travel axis are equal to each other regardless of the tilt angle $\theta$.

Fig. 5 shows the multi-row detector at the view angle $\phi$= $\pi$/2. A position h1($\pi$/2, i) of the ith channel of the first detector sequence (j = 1) relative to the linear travel axis and a position h2($\pi$/2, i) of the ith channel of the second detector sequence (j = -1) relative to the linear travel axis are not equal to each other because of the tilt angle $\theta$.

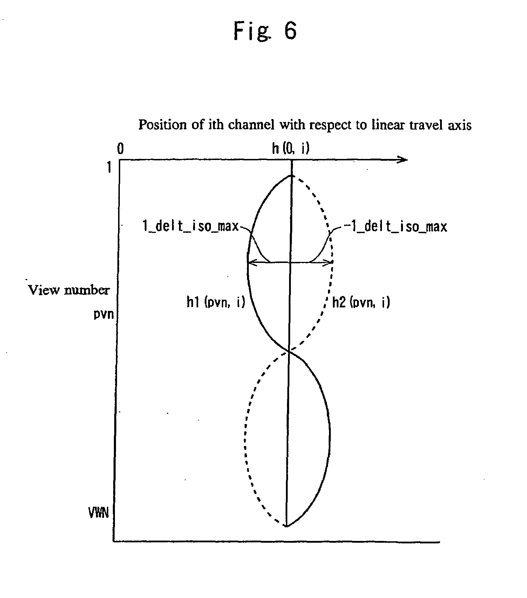

Fig. 6 shows the manner in which a position h1(pvn, i) of the ith channel of the first detector sequence (j = 1) relative to the linear travel axis and a position h2(pvn, i) of the ith channel of the second detector sequence (j = -1) relative to the linear travel axis vary every views due to the inclination of the scan surface. When pvn is defined as the view number and set to 1≤pvn≤VWN, a position hj(pvn, i) is generally given by the following equation:

$$\text{hj(pvn, i) = h(0, i) + j\_delt\_iso\_max.sin(2\pi(pvn-1)/VMN\}}$$

Incidentally, the view angle $\phi$ = $2\pi$(pvn-1)/VWN

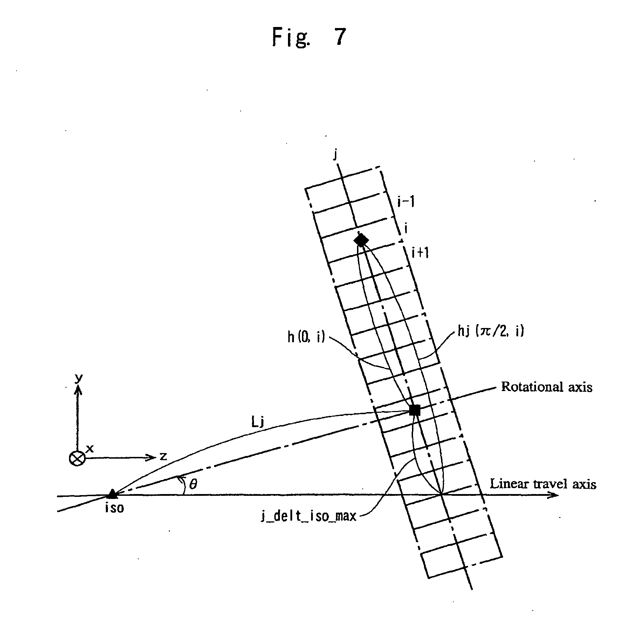

Fig. 7 shows a jth detector sequence at the view angle $\phi$= $\pi$/2. As is understood from the figure, the following equation is established:

$$\text{j\_delt\_iso\_max = Lj·tan}\theta$$

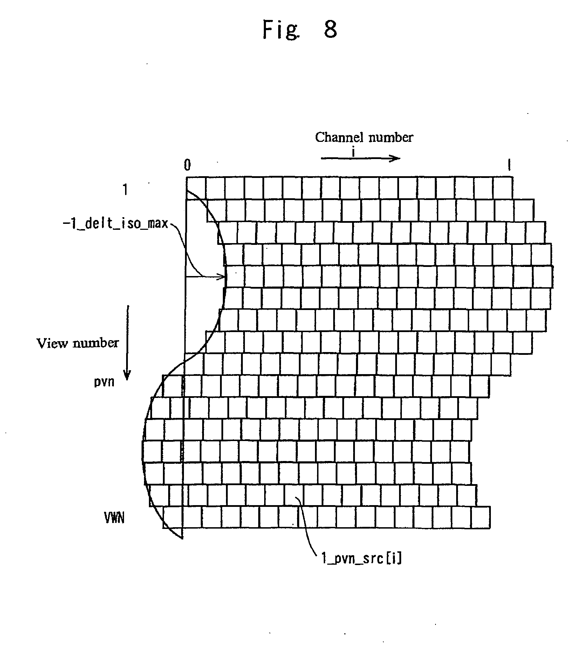

Fig. 8 is an explanatory diagram of the data position moving process about the first detector sequence (j = 1) of the multi-row detector. Data are arranged in the form of a two dimension of a channel number axis and a view number axis. Next, data positions of view numbers pvn are moved in a channel number direction by the following expression.

$$\text{-1\_delt\_iso\_max.sin\{2\pi(pvn-1)/VWN\}}$$

In a post-movement data array shown in Fig. 8, the positions of the data on straight lines as seen in the view number direction with respect to the linear travel axis become identical.

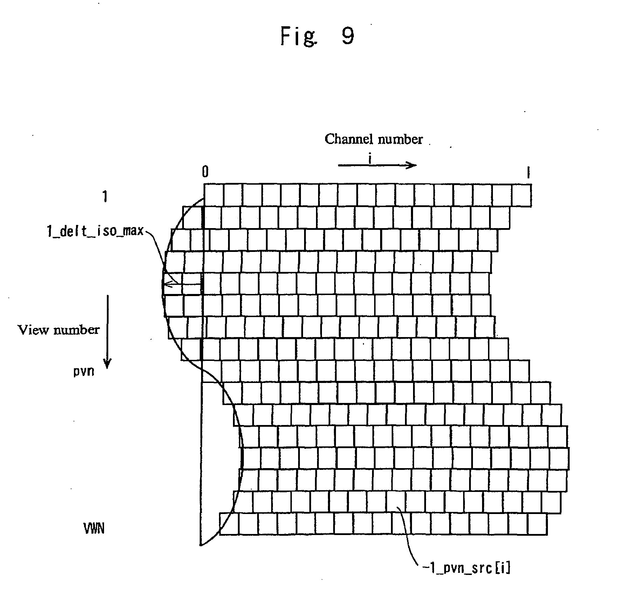

Fig. 9 is an explanatory diagram of a data position moving process about the second detector sequence (j = -1) of the multi-row detector. Data are arranged in the form of a two dimension of a channel number axis and a view number axis. Next, data positions of view numbers pvn are moved by the following expression.

$$\text{1\_delt\_iso\_max·sin\{2\pi(pvn-1)/VWN\}}$$

In a post-movement data array shown in Fig. 9, the positions of the data on the straight lines as seen in the view number direction with respect to the linear travel axis become identical.

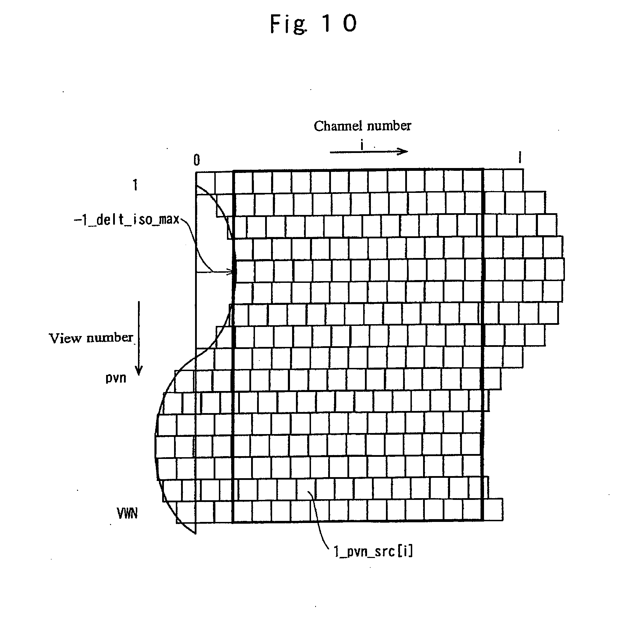

Fig. 10 is an explanatory diagram of a data cutting-out process about the first detector sequence (j = 1) of the multi-row detector. In a data array subsequent to the movement of the data positions, data lying in a range (within heavy-line frame) in which data exist over all views as seen in a view direction, are cut out.

A data cut-out range is generally expressed in the following manner. That is, when a channel-to-channel distance is defined as DMM and Roundup{} is defined as a round function, the data cut-out range extends from a (Roundup{Lj·tan$\theta$/DMM}+1)th channel of a pvnth view of a jth detector sequence to an (I-Roundup{Lj·tan$\theta$/DMM}-1)th channel.

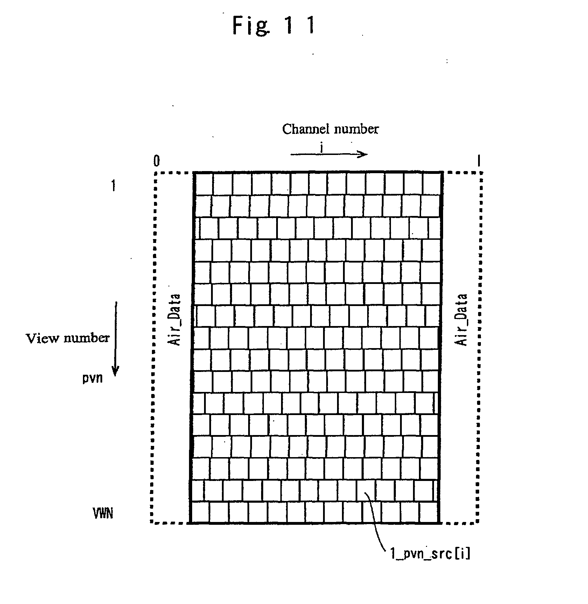

Fig. 11 is an explanatory diagram of a dummy data adding process about the first detector sequence (j = 1) of the multi-row detector. Air data are added to a cut-out data array to fix up a data range in a manner similar to the first data array.

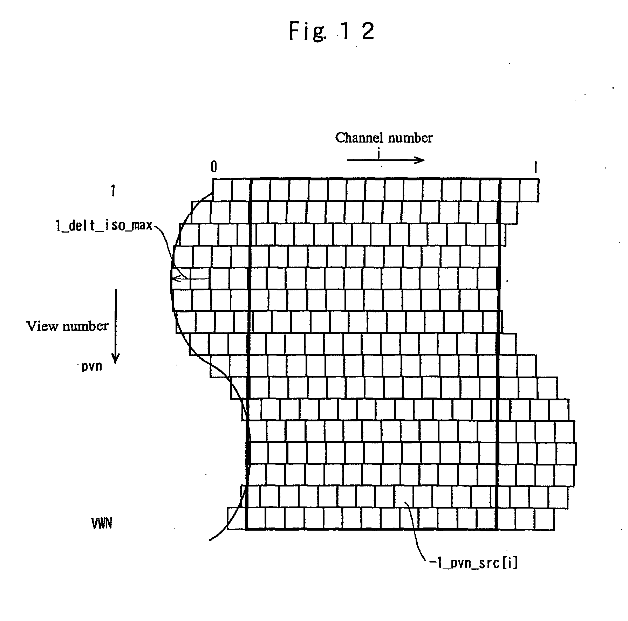

Fig. 12 is an explanatory diagram of a data cutting-out process about the second detector sequence (j = -1) of the multi-row detector. In a data array subsequent to the movement of data positions, data lying in a range (within heavy-line frame) in which data exist over all views as seen in a view direction, are cut out.

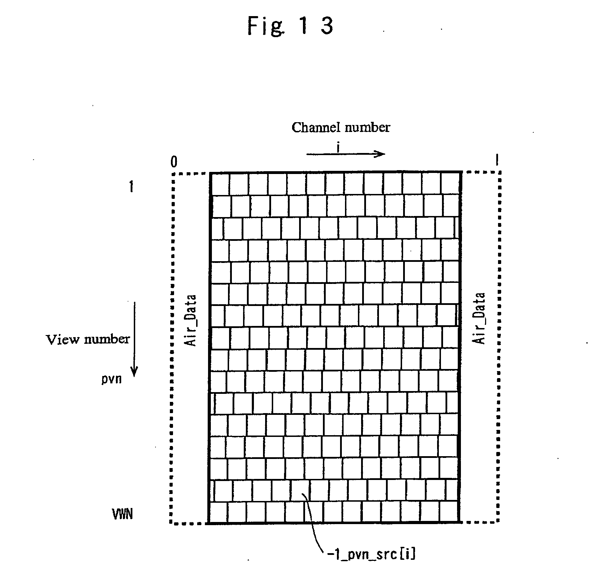

Fig. 13 is an explanatory diagram of a dummy data adding process about the second detector sequence (j = -1) of the multi-row detector. Air data are added to a cut-out data array to arrange or fix up a data range in a manner similar to the first data array.

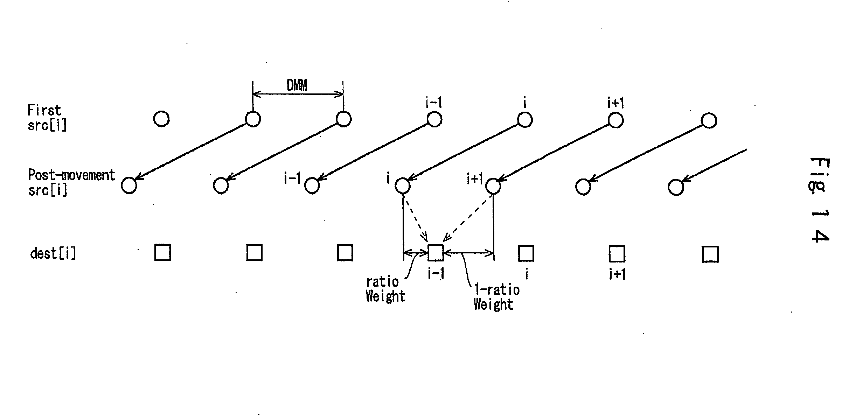

Fig. 14 is an explanatory diagram of a data converting process based on a linear interpolating computation. Src{i} indicate data of respective channels in a first data array at a given view.

The positions of the data are shifted due to a data position moving process. Therefore, data dest[i] at the first position is determined by a linear interpolating process.

When the data are moved in the direction of a small channel number as shown in Fig. 14 assuming that int{} is defined as an integral-part taking-out function and abs{} is defined as an absolute value function, the linear interpolating process results in the following equations:

$$delt\_iso = delt\_iso\_max \cdot \sin\{2\pi(pvn-1)/VWN\}$$

$$int\_delt\_iso = abs\{int\{delt\_iso/DMM\}\}$$

$$ratio = abs\{delt\_iso/DMM\} - int\_delt\_iso$$

$$dest\{i - int\_delt\_iso\} = src\{i\} \cdot (1 - ratio) + src\{i+1\} \cdot ratio$$

On the other hand, when the data are moved in the direction of a large channel number, the linear interpolating process results in the following equation:

$$dest\{i + int\_delt\_iso\} = src\{i\} \cdot (1 - ratio) + src\{i+1\} \cdot ratio$$

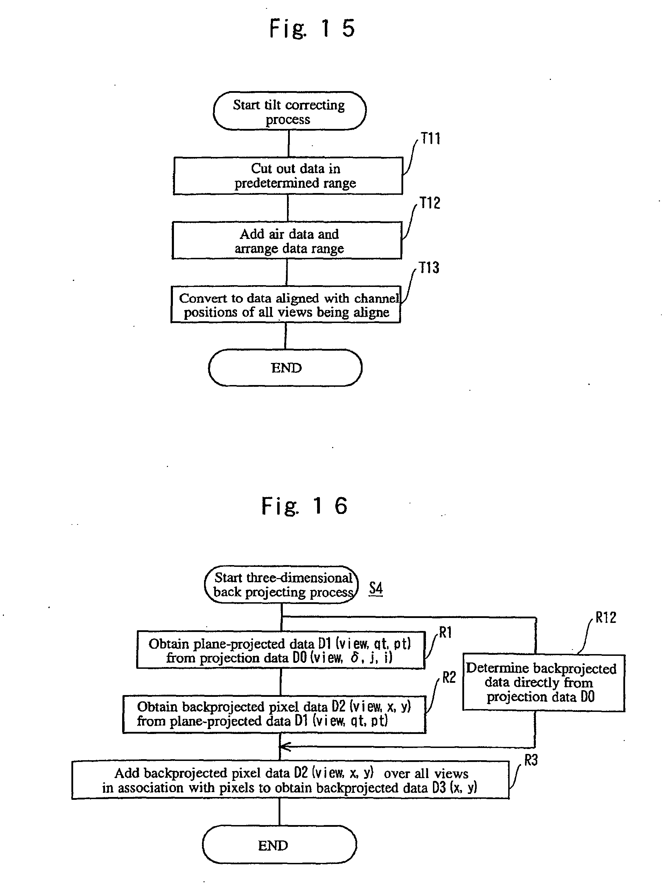

Fig. 15 is a flowchart of another example of the tilt correcting process. In Step T11, data from a (Roundup$\{Lj \cdot \tan\theta/DMM\}+1$)th channel of a pvnth view of a jth detector sequence to an (I-Roundup$\{Lj \cdot \tan\theta/DMM\}-1$)th channel are cut out. In Step T12, dummy data is added to the cut-out data to arrange or fix up a data range of each view. In Step T13, data positions are moved to make a two-dimensional data array as shown in Fig. 11 or 13. The data are converted to data in which channel positions of all views are aligned with one another. This example is one in which the data position moving process has been made after the dummy data adding process. Incidentally, the data position moving process is done after the data cutting-out process and thereafter the dummy data adding process may be carried out.

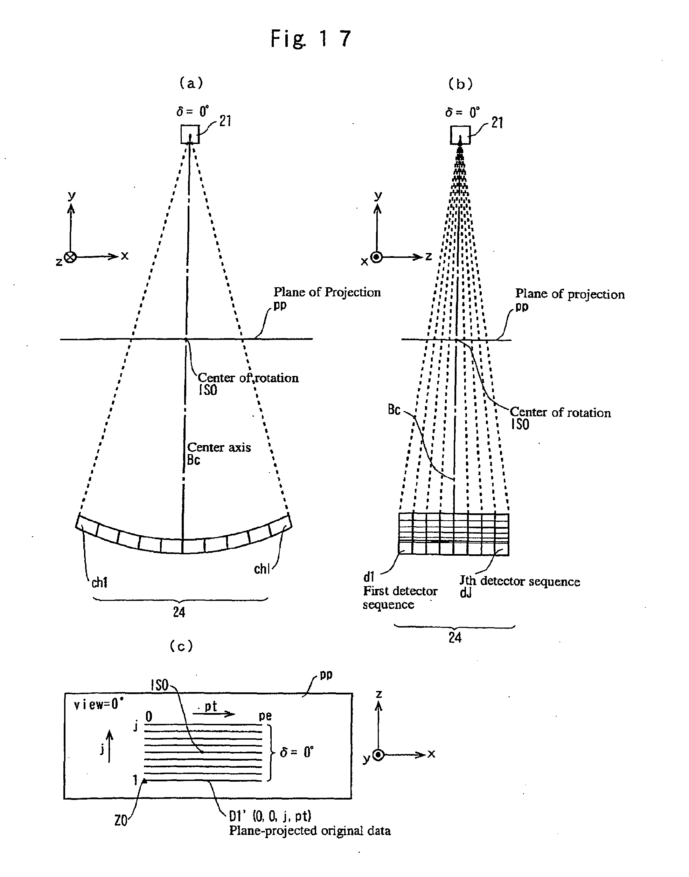

The three-dimensional back projecting process will be explained. Fig. 16 is a detailed flowchart of the three-dimensional back projecting process (S4). In Step R1, plane-projected data D1(view, qt, pt) are obtained from projection data D0(view, $\delta$, j, i). This process will be described later with reference to Figs. 17 through 25.

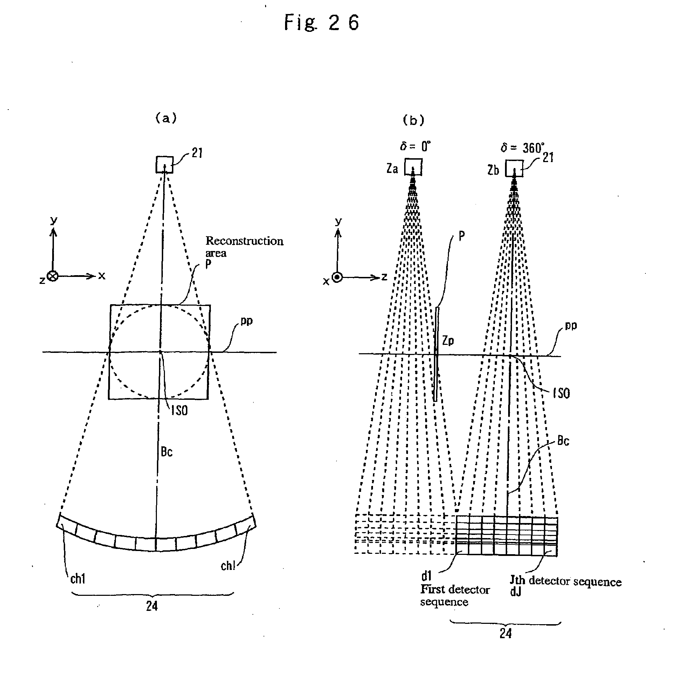

In Step R2, backprojected pixel data D2(view, x, y) are obtained from the data D1(view, qt, pt) plane-projected onto the plane of projection. This process will be described later with reference to Figs. 26 through 31 and Figs. 33 through 38. Alternatively, the backprojected pixel data D2 may be determined directly from the projection data D0 like Step R12.

In Step R3, views corresponding to 360° are added to the backprojected pixel data D2(view, x, y) in association with pixels, or views corresponding to "180° + fan angle" are added thereto to obtain backprojected data D3(x, y). This process will be explained later with reference to Fig. 32.

Figs. 17(a) and 17(b) show the layout of the X-ray tube 21 and the multi-row detector 24 at view = 0° and $\delta$ = 0°. A plane pp of projection at this time is an xz plane that passes through the center of rotation ISO. The xz plane is inclined to the linear travel axis. The axis of rotation of a scan is placed on the xz plane.

Projection data D0(view = 0, $\delta$ = 0, j, i) obtained at respective channels of the multi-row detector 24 are multiplied by distance coefficients and laid out at positions where the respective channels are plane-projected onto the projection plane pp as viewed in an X-ray penetration direction, followed by interpolating processing in a channel direction to thereby make data densities high sufficiently, whereby plane-projected data D1'(view = 0, $\delta$ = 0, j, pt) are obtained as shown in Fig. 17(c). This will be expressed as "the projection data D0(view, $\delta$, j, i) are plane-projected onto the projection plane pp in the X-ray penetration direction".

Incidentally, when the distance from an X-ray focal point of the X-ray tube 21 to each channel of the multi-row detector 24 is defined as r0, and the distance from the X-ray tube 21 to the position of projection on the projection plane pp is defined as r1, the distance coefficient is given as $(r1/r0)^2$. Z0 shown in Fig. 17(c) indicates origin coordinates indicative of a spatial position of plane-projected data D1'(view = 0, δ = 0, j = 1, pt = 0).

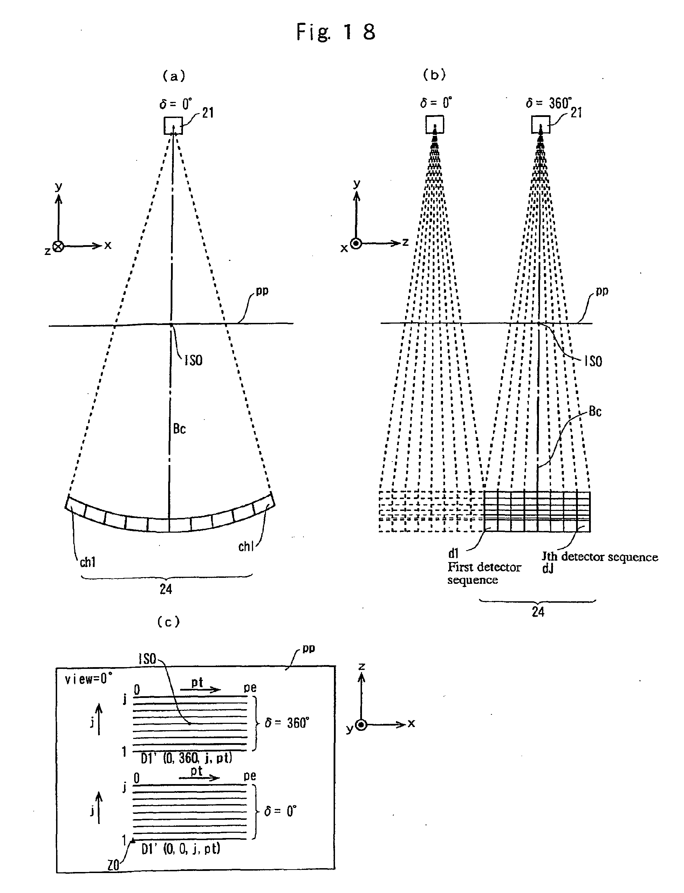

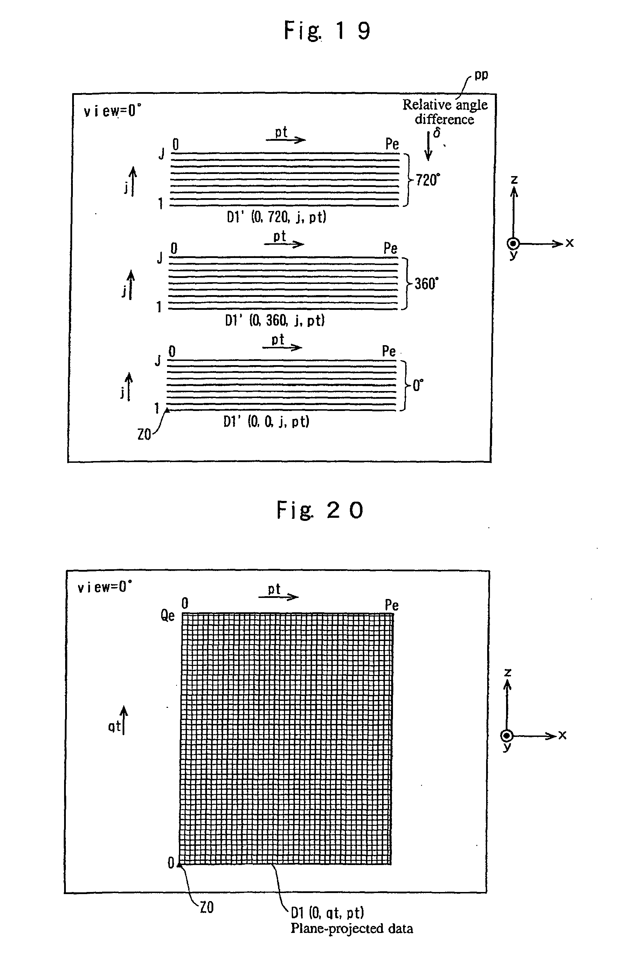

Figs. 18(a) and 18(b) show the layout of the X-ray tube 21 and the multi-row detector 24 at view = 0° and δ = 360° (i.e., after one rotation from δ = 0°). When projection data D0(view = 0, δ = 360, j, i) obtained at this time are plane-projected onto the projection plane pp, plane-projected data D1'(view = 0, δ = 360, j, pt) are obtained as shown in Fig. 18(c). Similarly, plane-projected data D1'(view = 0, δ = 720, j, pt) corresponding to view = 0° and δ = 720° (third rotation) are also obtained as shown in Fig. 19.

Next, such plane-projected data D1'(0, 0, j, i), D1'(0, 360, j, i) and D1'(0, 720, j, i) as shown in Fig. 19 are subjected to an interpolation/extrapolation process to calculate data D1(view = 0, qt, pt) plane-projected sufficiently densely in a qt direction (corresponding to the direction orthogonal to a crossline of the reconstruction area P and the projection plane pp) and a pt direction (corresponding to the direction parallel to the crossline of the reconstruction area P and the projection plane pp). Here, the density of the plane-projected data D1(view = 0, qt, pt) may preferably be set sufficiently higher than a pixel density in the reconstruction area such that the interpolation process is omitted when backprojected pixel data D2 is determined from the plane-proj ected data D1.

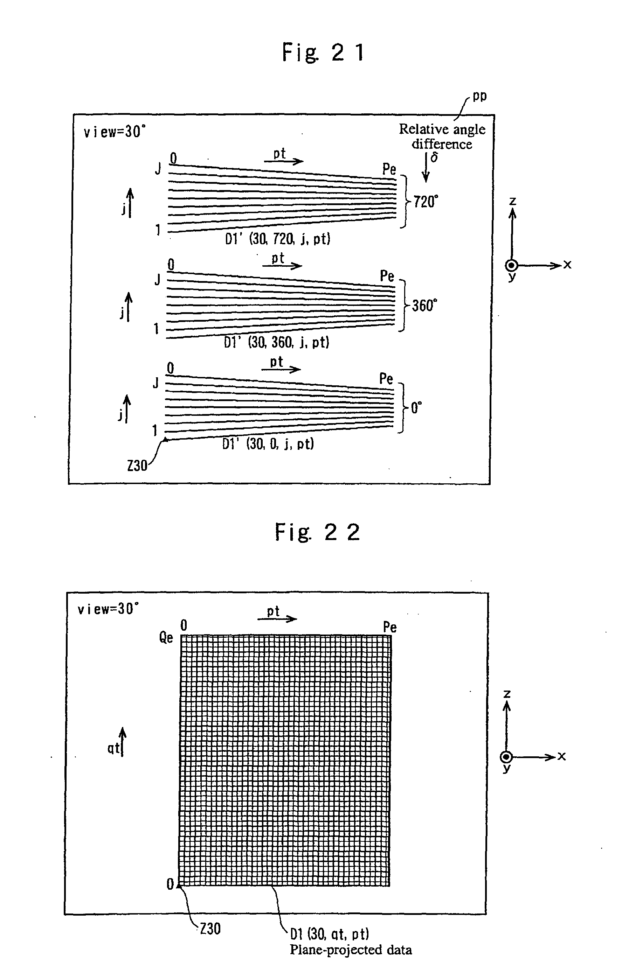

Fig. 21 is a conceptual diagram of plane-projected data D1'(view = 30, δ = 0, j, pt), D1'(view = 30, δ = 360, j, pt) and D1'(view = 30, δ = 720, j, pt) corresponding to a first rotation, a second rotation and a third rotation respectively.

As compared with view = 0°, the first channel side of the multi-row detector 24 approaches the projection plane pp and the Ith channel side becomes distant from the projection plane pp. Therefore, the plane-projected data D1'(30, 0, j, pt), D1'(30, 360, j, pt) and D1'(30, 720, j, pt) become wide on the first channel side, whereas they become narrow on the Ith channel side. Incidentally, Z30 indicates origin coordinates indicative of a spatial position of plane-projected data D1'(30, 0, 1, 0).

Fig. 22 is a conceptual diagram of plane-projected data D1(30, qt, pt) calculated sufficiently densely in a qt direction and a pt direction by effecting an interpolation/extrapolation process on the plane-projected data D1'(30, 0, j, pt), D1'(30, 360, j, pt) and D1'(30, 720, j, pt).

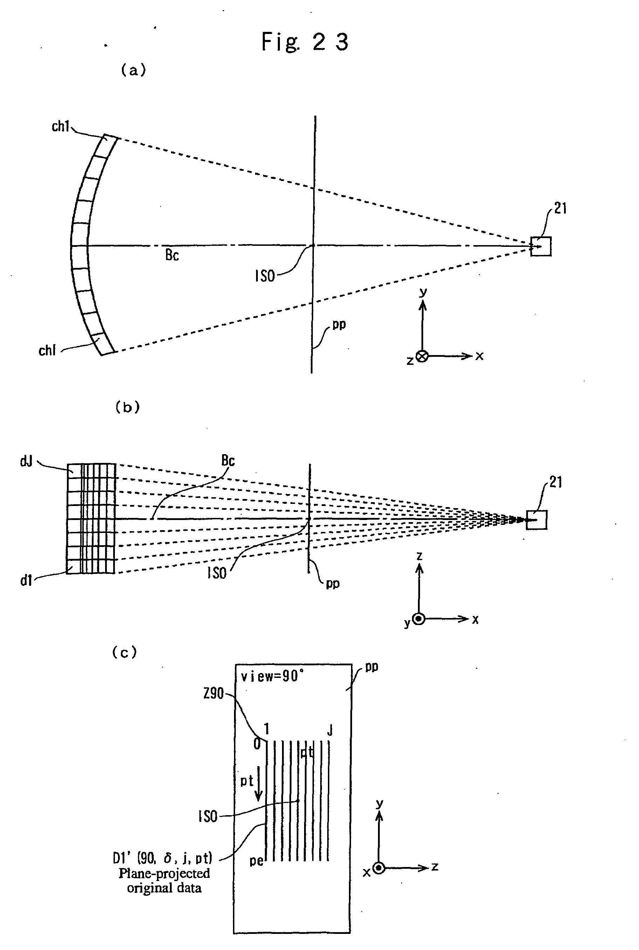

Figs. 23(a) and 23(b) show the layout of the X-ray tube 21 and the multi-row detector 24 at view = 90°. A projection plane pp at this time is a yz plane that passes through the center of rotation ISO. When obtained projection data D0(view = 90, δ, j, i) are plane-projected onto the projection plane pp, plane-projected data D1'(view = 90, δ, j, pt) are obtained as shown in Fig. 23(c).

Thus, the xz plane that passes through the center of rotation ISO is defined as the projection plane pp in -45° ≤view< 45° or a view angular range containing even a periphery with this view as a principal part, and 135° ≤view< 225° or a view angular range containing even a periphery with this view as a principal part. The yz plane that passes through the center of rotation ISO is defined as the projection plane pp in 45° ≤view< 135° or a view angular range containing even a periphery with this view as a principal part, and 225° ≤view< 315° or a view angular range containing even a periphery with this view as a principal part.

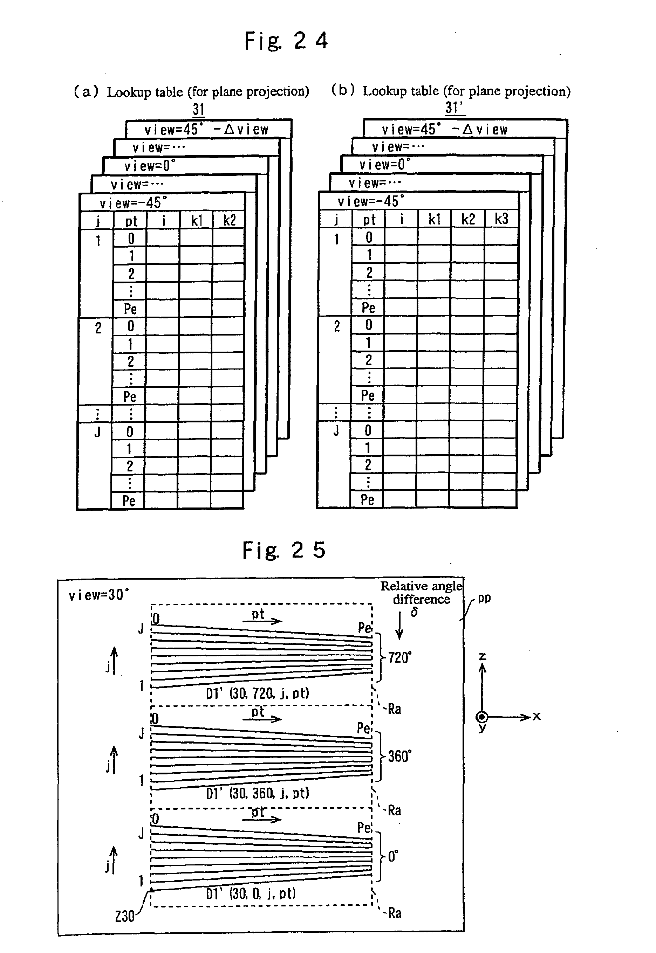

It is preferable that when the plane-projected data D1'(view, δ, j, pt) are determined from the projection data D0(view, δ, j, i), such a plane-projecting lookup table 31 as shown in Fig. 24 is stored in the memory device 7 and utilized.

The lookup table 31 shown in Fig. 24(a) is one for determining the plane-projected data D1'(view, δ, j, pt) by two-point interpolation/extrapolation. Every view angles views in the view angular range of -45° ≤view< 45° (or view angular range containing even the periphery with this view as the principal part), a plurality of channel addresses i for determining plane-projected data D1'(view, δ, j, pt) at coordinates (j, pt) by the two-point interpolation/extrapolation, reference channel addresses i for extracting i+1 projection data D0, and two-point interpolation/extrapolation factors or coefficients k1 and k2 in a pt direction are calculated and set in advance.

The data D 1 are expressed as follows:

D1(view,δ,j,pt)=k1×D0(view,δ,j,i)+k2×D0(view,δ,j, i+1). Incidentally, Δview indicates a step angle (view angle difference between adjacent views) for each view angle. Δview results in "0.36°" in the case of 1000 views in total, for example.

The lookup table 31' shown in Fig. 24(b) is one for determining the plane-projected data D1'(view, qt, pt) by three-point interpolation/extrapolation. Every view angles views in the view angular range of -45° ≤view< 45° (or view angular range containing even the periphery with this view as the principal part), a plurality of channel addresses i for determining plane-projected data D1'(view, δ, j, pt) at coordinates (j, pt) by the three-point interpolation/extrapolation, reference channel addresses i for extracting i+1 and i+2 projection data D0, and three-point interpolation/extrapolation coefficients k1, k2 and k3 in a pt direction are calculated and set in advance.

In the case of a helical scan, interpolation coefficients in a qt direction are also set to lookup tables similar to the lookup tables 31 and 31'. Similar interpolation/extrapolation is effected even in the qt direction. The interpolation in the qt direction is repeated in such rectangular areas Ra as shown in Fig. 25. The interpolation is symmetric within the rectangular area Ra as viewed in the qt direction with a center line interposed therebetween. Incidentally, interpolation/extrapolation is made within such one rectangular area Ra as shown in Fig. 25 in the case of an axial scan.

Even other than the view angular range of -45° ≤view< 45° (or view angular range containing even the periphery with this view as the principal part) from geometrical similarity, the lookup tables 31 and 31' in the view angular range of -45° ≤view< 45° (or view angular range containing even the periphery with this view as the principal part) can be appropriated.

Fig. 26 illustrates a spatial position of a reconstruction area P as an example. The present drawing shows an example in which when a z coordinate of the X-ray tube 21 at view = 0° and δ = 0° is defined as Za and a z coordinate of the X-ray tube 21 at view = 0° and δ = 360° is defined as Zb, the reconstruction area P exists in the position of Zp = Za + (Zb - Za)/4.

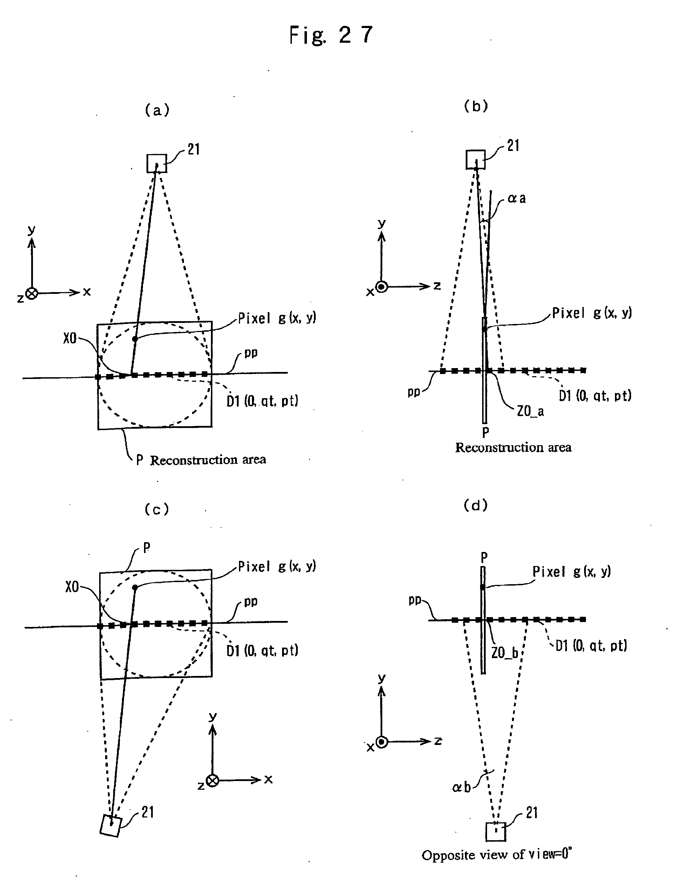

Fig. 27 shows a state in which plane-projected data D1(0, qt, pt) are projected onto a reconstruction area P in an X-ray penetration direction to determine backprojected pixel data D2(0, x, y). As shown in Fig. 27(a), a coordinate X0 is determined from a point where a straight line connecting the focal point of the X-ray tube 21 at view = 0° and a pixel g(x, y) on the reconstruction area P intersects a projection plane pp.

As shown in Fig. 27(b) as well, a coordinate Z0_a is determined from a point where a straight line connecting the focal point of the X-ray tube 21 at view = 0° and a pixel g(x, y) on the reconstruction area P intersects the projection plane pp. Similarly, as shown in Figs. 27(c) and 27(d), a coordinate Z0_b is determined from a point where a straight line connecting the focal point of the X-ray tube 21 at an opposite view and a pixel g(x, y) on the reconstruction area P intersects a projection plane pp.

Incidentally, when the angle which a straight line connecting the focal point of the X-ray tube 21 and a pixel g(x, y) on a reconstruction area P at view = βa forms with a center axis Bc of an X-ray beam is defined as γ, and its opposite view is defined as view = βb, the following equation is established:

$$\beta b = \beta a + 180° - 2\gamma$$

Next, plane-projected data D1(0, qt_a, pt) corresponding to the coordinates (X0, Z0_a) is determined. Also plane-projected data D1(0, qt_b, pt) corresponding to the coordinates (X0, Z0_b) is determined. When the distance from the X-ray focal point of the X-ray tube 21 at view = 0° to the plane-projected data D1(0, qt_a, pt) is set as r0_0a, and the distance from the X-ray focal point of the X-ray tube 21 to the pixel g(x, y) is assumed to be r0_1a, backprojected pixel data D2(0, x, y) a at view = 0° is determined from the following equation:

$$D2(0, x, y)\_a = (r0\_0a/r0\_1a)^2 \cdot D1(0, qt\_a, pt)$$

When the distance from the X-ray tube 21 at an opposite view to the plane-projected data D1(0, qt_b, pt) is defined as r0_0b, and the distance from the X-ray tube 21 to the pixel g(x, y) is defined as r0_1b, backprojected pixel data D2(0, x, y)_b of the opposite view is determined from the following equation:

$$D2(0, x, y)\_b = (r0\_0b/r0\_1b)^2 \cdot D1 (0, qt\_b, pt)$$

Next, the backprojected pixel data D2(0, x, y)_a and D2(0, x, y)_b are multiplied by cone beam reconstruction weighting factors ωa and ωb dependent on angles αa and αb shown in Fig. 27 and then added together to thereby determine backprojected pixel data D2(0, x, y).

$$D2(0, x, y) = \omega a \cdot D2(0, x, y)\_a + \omega b \cdot D2(0, x, y)\_b$$

Incidentally, the angle αa indicates an angle which an X ray that passes through the pixel g(x, y) at view = 0° forms with a plane containing a reconstruction area P. Also the angle αb indicates an angle which an X ray that passes through the pixel g(x, y) at an opposite view forms with a plane containing a reconstruction area P. Further, the following equation is established:

$$\omega a + \omega b = 1$$

Cone angle archfacts can be reduced by multiplying the data by the cone beam reconstruction weighting factors ωa and ωb and adding the same together. As the cone beam reconstruction weighting factors ωa and ωb, for example, ones determined from the following equations can be used.

When max[] is defined as a function that takes a large value, and 1/2 of a fan beam angle is defined as ymax, the following are expressed as follows:

$$ga = \max[0, \{(\pi/2 + \gamma max) - |\beta a|\}] \cdot |\tan(\alpha a)|$$

$$gb = \max[0, \{\pi/2 + \gamma max) - |\beta b|\}] \cdot |\tan(\alpha b)|$$

$$xa = 2 \cdot ga^q/(ga^q + gb^q)$$

$$xb = 2.gb^q/(ga^q + gb^q)$$

$$\omega a = xa^2 \cdot (3 - 2xa)$$

$$\omega b = xb^2 \cdot (3 - 2xb)$$

$$(e.g., q = 1)$$

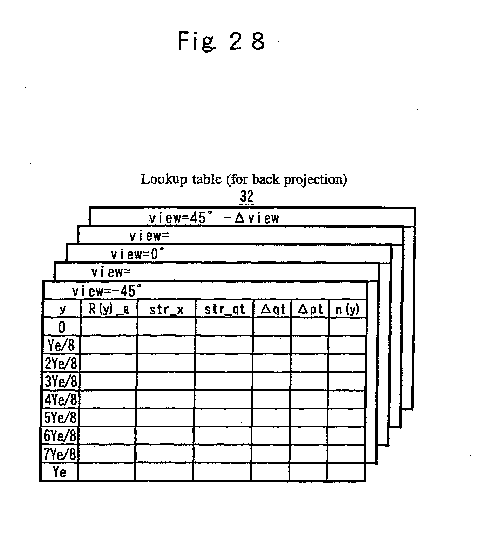

Fig. 28 is a conceptual diagram of a back projecting lookup table 32 stored in the memory device 7. Backprojected pixel data D2(view, x, y)_a of respective pixels constituting a plurality of lines (y = 0, Ye/8, 2Ye/8, 3Ye/8, 4Ye/8, 5Ye/8, 6Ye/8, 7Ye/8, Ye) corresponding to plural (nine in the present drawing) lines on a reconstruction area P with plural pixel intervals defined thereamong and parallel to the plane of projection (x direction in the drawing) are determined using the back projecting lookup table 32.

Weights $R(y)\_a = (r0\_0a/r0\_1a)^2$, start addresses str_x and str_qt, sampling pitches $\Delta qt$ and $\Delta pt$, and the number of samplings n(y) are calculated and set to the lookup table 32 in advance as conversion computational parameters for determining one backprojected pixel data D2(view, x, y)_a from a y coordinate y (y coordinate of each line) of backprojected pixel data D2 and one plane-projected data D1(view, qt, pt), every view angles views in a view angular range of -45° ≤view< 45° (or view angular range containing even a periphery with the view as a principal part).

Even other than the view angular range of -45° ≤view< 45° (or view angular range containing even the periphery with this view as the principal part) from geometrical similarity, the lookup table 32 in the view angular range of -45° ≤view< 45° (or view angular range containing even the periphery with this view as the principal part) can be used.

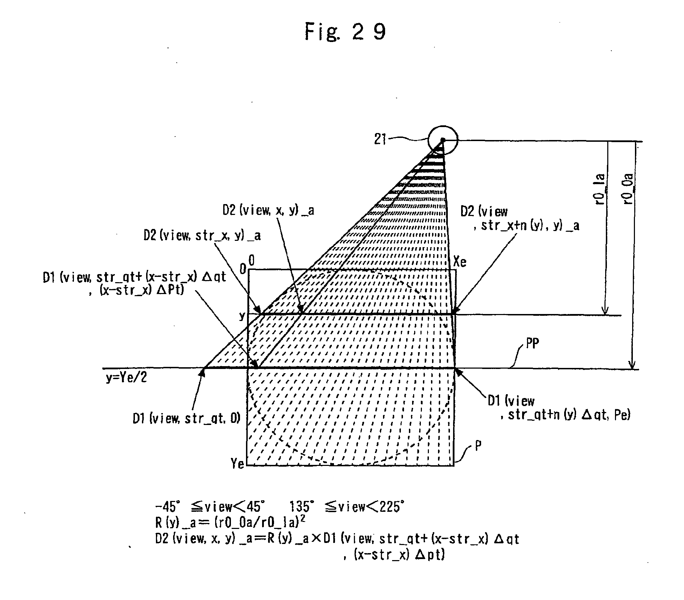

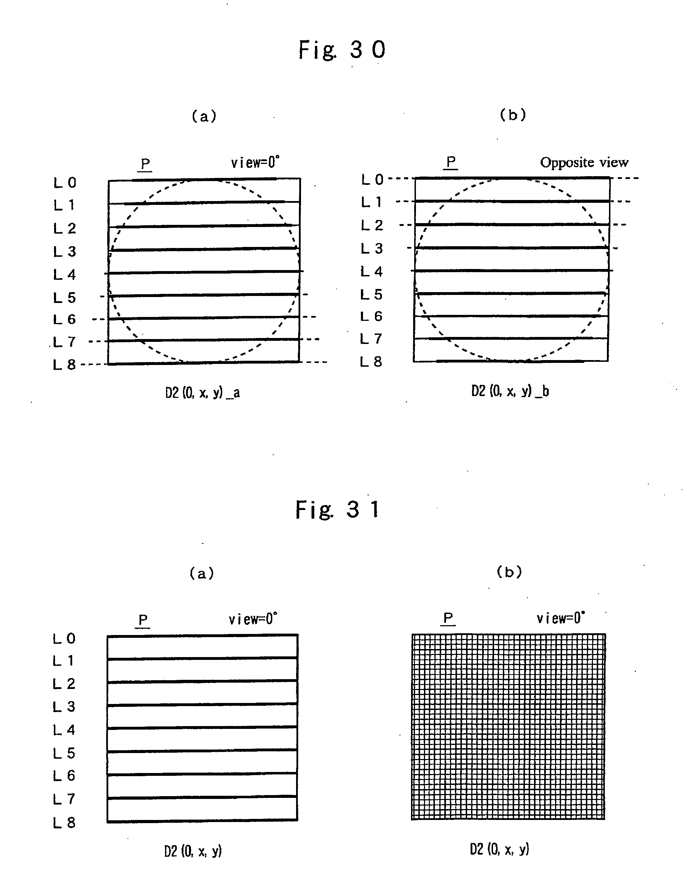

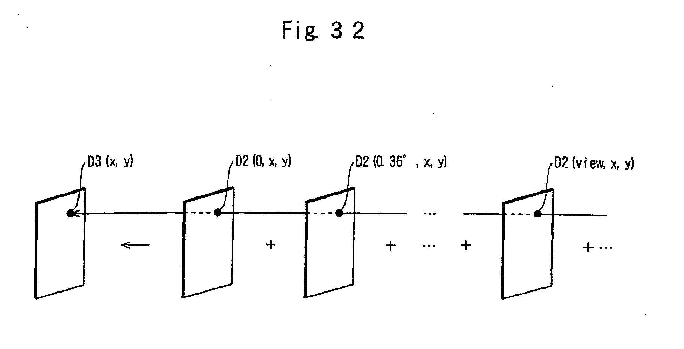

Fig. 29 shows the situation in which when a reconstruction area P is a plane parallel to an xy plane and the plane pp of projection is an xz plane, backprojected pixel data D2(view, str_x, y)_a through D2(view, str_x+n(y), y)_a about a pixel g(x, y) lying on lines parallel to an x axis are determined.

Weights R(y)_a about the pixel g(x, y) lying on the lines parallel to the x axis all assumes $(r0\_1a/r0\_0a)^2$ and become common. Thus, the following equation is represented:

$$D2(view, x, y)\_a = R(y)\_a \times D1(view, str\_qt + (x - str\_x)\Delta qt, st\_pt + (x - str\_x)\Delta pt)$$

Fig. 30(a) is a conceptual diagram showing backprojected pixel data D2(view = 0, x, y)_a about lines L0 through L8 parallel to an x axis. Fig. 30(b) is a conceptual diagram showing backprojected pixel data D2(view = 0, x, y)_b obtained in like manner, about lines L0 through L8 parallel to an x axis.

Fig. 31(a) is a conceptual diagram showing backprojected pixel data D2(0, x, y) determined by multiplying backprojected pixel data D2(0, x, y)_a and D2(0, x, y)_b by cone beam reconstruction weighting factors ωa and ωb and adding the same. Fig. 31(b) is a conceptual diagram showing backprojected pixel data D2(0, x, y) determined by interpolating among lines L0 through L8.

Fig. 32 shows a state in which the backprojected pixel data D2(view, x, y) shown in Fig. 31(b) are added corresponding to pixels over all views to obtain backprojected data D3(x, y). That is,

$$D3(x, y) = {}_{view}\Sigma D2(view, x, y)$$

According to the X-ray CT apparatus 100 that performs such operation, data D1 plane-projected from projection data D0 are determined, and the plane-projected data D1 are projected onto a reconstruction area in an X-ray penetration direction to determine backprojected pixel data D2. Therefore, it is possible to perform reconstruction using projection data properly corresponding to an X beam transmitted through the reconstruction area. Thus, an image excellent in quality can be obtained. Looking overall, processing can be simplified and speeded up.

Further, when the backprojected pixel data D2 are determined from the plane-projected data D1, only backprojected pixel data D2 on respective pixels constituting lines L0 through L8 are determined and a process for interpolating among the lines is performed. Therefore, a processing time interval can be shortened as compared with the determination of the backprojected pixel data D2 on all pixels constituting the reconstruction area P from the plane-projected data D1.

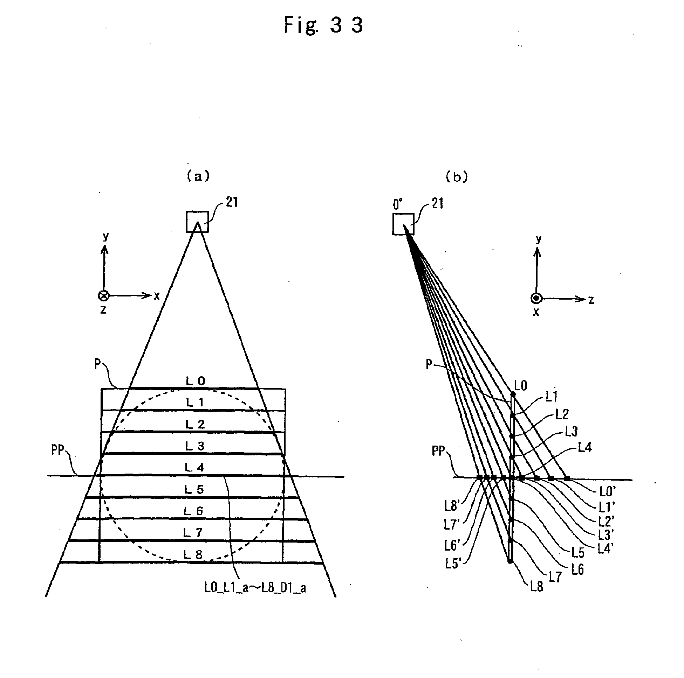

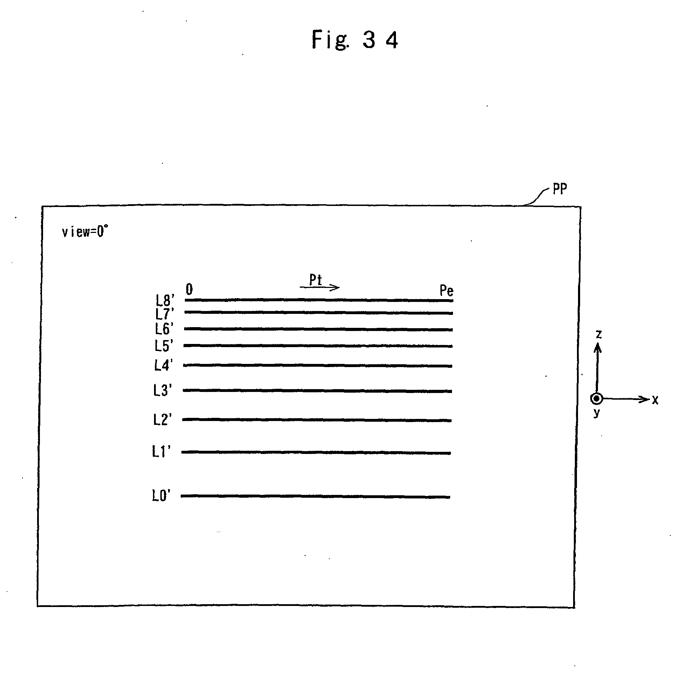

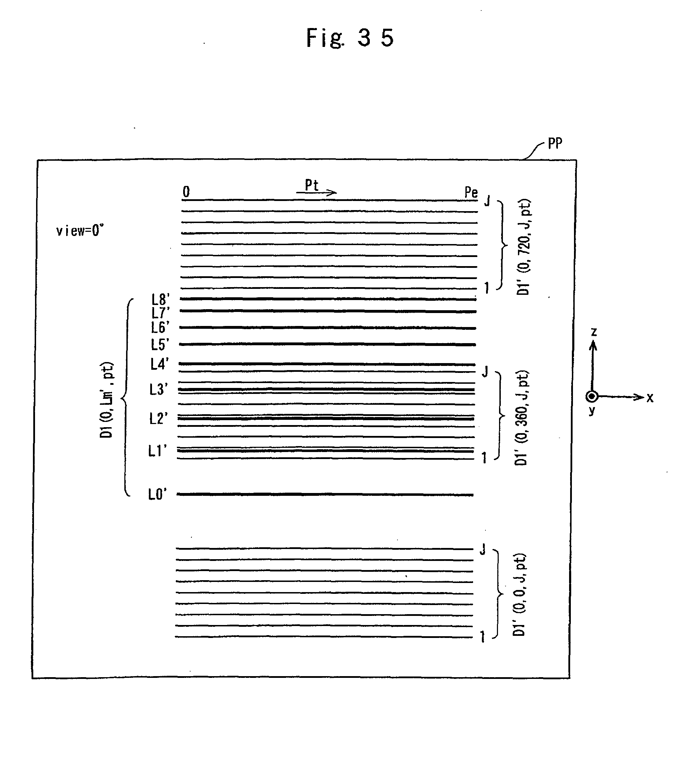

Another example of the best mode for carrying out the present invention will be explained. As shown in Figs. 33 and 34, lines on a projection plane pp, corresponding to lines L0 through L8 on a reconstruction area P are assumed to be L0' through L8'.

As shown in Fig. 35, only plane-projected data D1(view, Lm', pt) on lines L0' through L8' are determined by an interpolation/extrapolation process on the basis of the plane-projected data D1'(view, $\delta$, j, pt) shown in Fig. 19. That is, plane-projected data D1(view, Lm', pt) on the lines L0' through L8' are determined from the projected data D0(view, $\delta$, j, i) in Step R1 of Fig. 16.

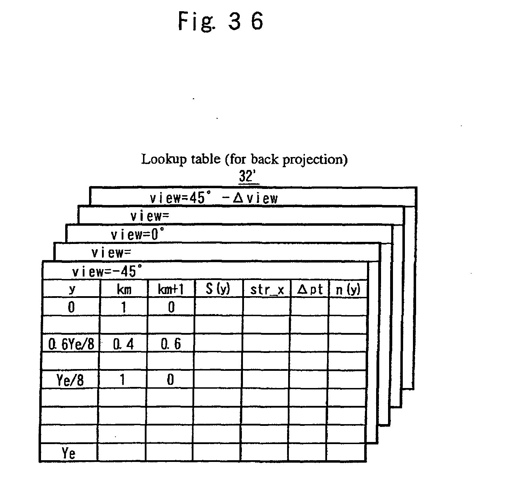

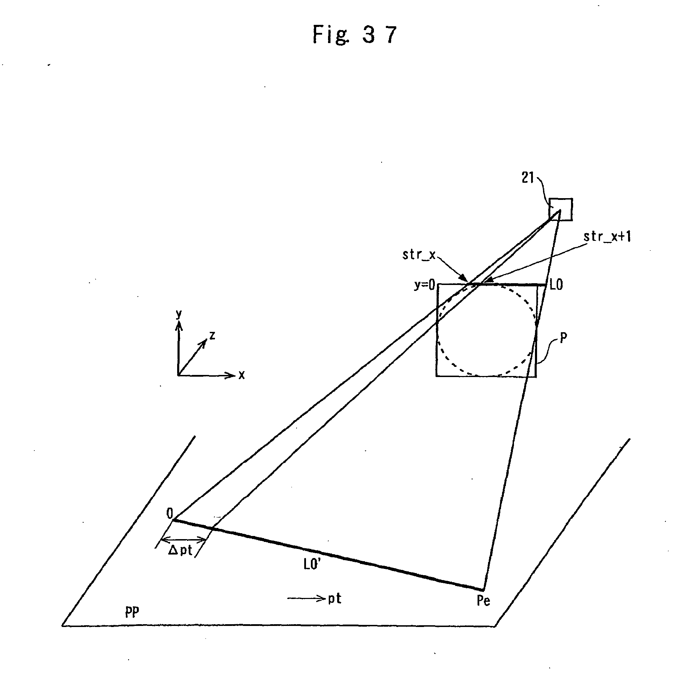

Next, backprojected pixel data D2(view, x, y) are determined using a back projecting lookup table 32' shown in Fig. 36. That is, the backprojected pixel data D2(view, x, y) are obtained from the data D1(view, Lm', pt) in Step R2 of Fig. 16.

Interpolation coefficients km and km+1, weights $S(y) = \omega a \times R(y)\_a$, a start address str_x, a sampling pitch $\Delta pt$, and the number of samplings n(y) are calculated and set to the lookup table 32' in advance as conversion computational parameters for determining one backprojected pixel data D2(view, x, y) from y coordinates y (y coordinates of all lines constituting reconstruction area P) of backprojected pixel data D2, and plane-projected data D1(view, Lm', pt) and D1(view, Lm+1', pt) of 2 lines, every view angles views in a view angular range of $-45° \leq view < 45°$ (or view angular range containing even a periphery with the view as a principal part).

Even other than the view angular range of $-45° \leq view < 45°$ (or view angular range containing even the periphery with this view as the principal part) from geometrical similarity, the lookup table 32 of the view angular range of $-45° \leq view < 45°$ (or view angular range containing even the periphery with this view as the principal part) can be used.

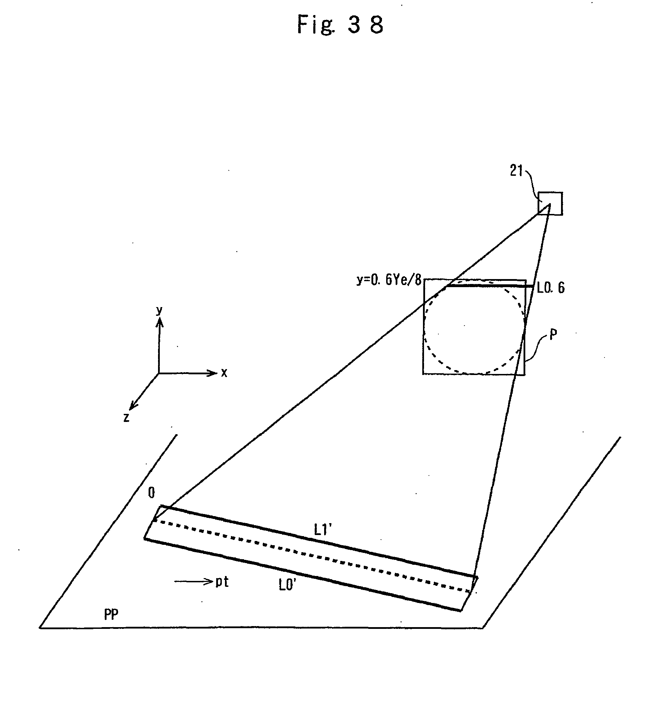

As shown in Fig. 37, backprojected pixel data D2(view, x, y = 0) can be determined by sampling plane-projected data D1(view, L0', pt) every $\Delta pt$. As shown in Fig. 38, backprojected pixel data D2(view, x, y = 0.6Ye/8) can be determined by effecting an interpolation process on plane-projected data D1(view, L0', pt) and D1(view, L1', pt).

$$D2(view, x, y) = S(y)$$

$$\times \{km \times D1(view, Lm', (x-str\_x)\Delta pt)$$

$$+ km+1 \times D1(view, Lm+1', (x-str\_x) \Delta pt)\}$$

According to this example, since only the plane-projected data D1(view, Lm', pt) on the lines L0' through L8' are determined, a processing time interval can be shortened as compared with the case in which a large quantity of plane-projected data D1(view, qt, pt) are determined.

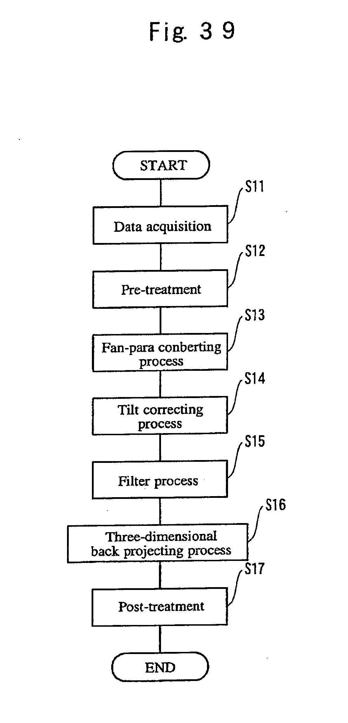

A further example of the best mode for carrying out the present invention will be explained. Fig. 39 is a flowchart showing a schematic flow of the operation of an X-ray CT apparatus 100. In Step S11, projection data D0f(view, $\delta$, j, i) corresponding to fan data represented by a view angle view, a relative angle difference $\delta$, a detector sequence number j and a channel number i are collected while an X-ray tube 21 and a multi-row detector 24 are being rotated aslant about an object to be imaged and a cradle 12 is being linearly moved.

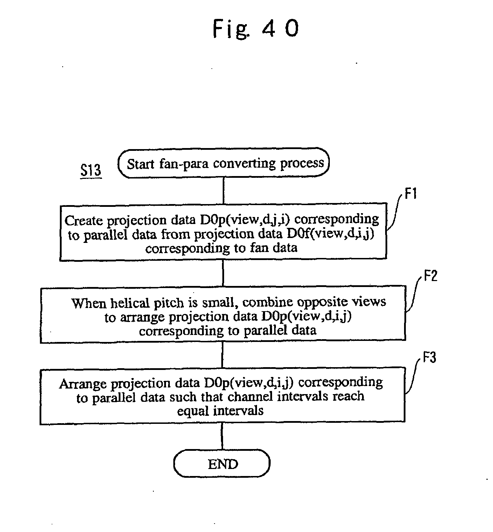

In Step S12, a pre-treatment (offset correction, logarithmic correction, X-ray dose correction and sensitivity correction) is effected on the projection data D0f(vie $\delta$, j, i) corresponding to the fan data. In Step S 13, a fan-para converting process is effected on the pre-treated projection data D0f(view, $\delta$, j, i) corresponding to the fan data to determine projection data D0p(view, $\delta$, j, i) corresponding to parallel data. The present fan-para converting process will be described later with reference to Fig. 40.

In Step S14, a tilt correcting process is performed. In Step S15, a filter process is effected on the tilt-processed projection data D0p(view, $\delta$, j, i) about the parallel data. That is, a Fourier transform is performed on the projection data, each of which is followed by being multiplied by a filter (reconstruction function) to perform an inverse Fourier transform thereof.

In Step S16, a three-dimensional back projecting process is effected on the projection data D0p(view, $\delta$, j, i)

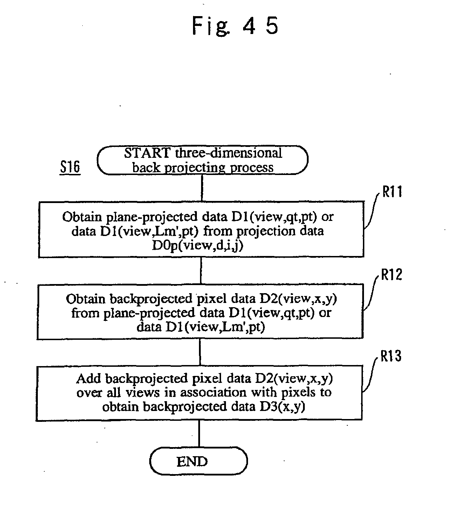

subjected to the filter process to determine backprojected data D3(x, y). The three-dimensional back projecting process will be explained later with reference to Fig. 45. In Step S17, a post-treatment is effected on the backprojected data D3(x, y) to obtain a CT image.

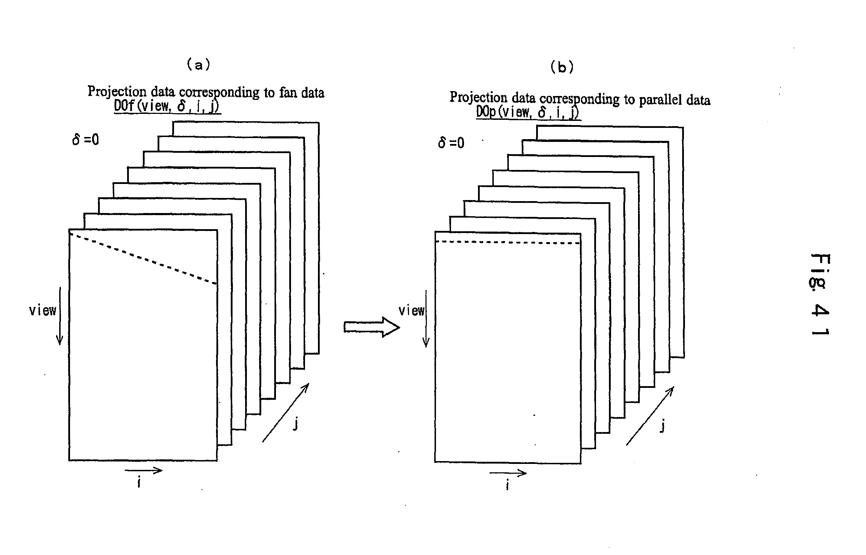

Fig. 40 is a detailed flowchart of the fan-para converting process (S13). In Step F1, projection data D0p(view, δ, j, i) corresponding to parallel data are created from projection data D0f(view, δ, i, j) corresponding to fan data. That is, the projection data D0f(view, δ, i, j) corresponding to the fan data are represented by sinograms as shown in Fig. 41(a). By picking up the data aslant as indicated by broken lines over the sinograms, the projection data D0p(view, δ, j, i) corresponding to the parallel data can be created as shown in Fig. 41 (b).

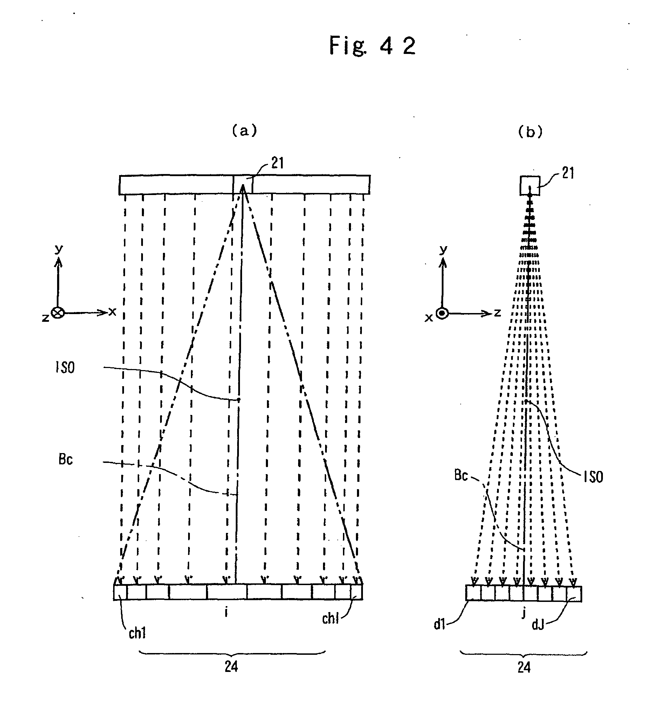

Penetration paths of X rays incident to respective channels corresponding to the projection data D0p(view, δ, j, i) corresponding to the parallel data are parallel in a channel direction as indicated by broken lines in Fig. 42(a) and become narrow in interval in the neighborhood of an end channel rather than a center channel. As indicated by broken lines in Fig. 42(b), the X-ray penetration paths are brought into radiation form with respect to a detector sequence direction.

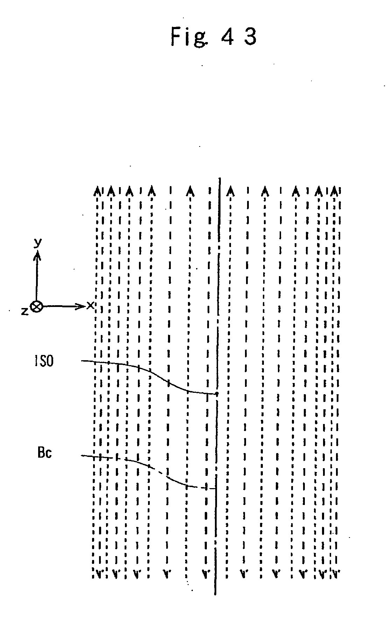

Referring back to Fig. 40, when a helical pitch is small, i.e., a contradiction related to a Z direction between data of opposite views is small, the opposite views are utilized in combination to bring the projection data D0p(view, δ, i, j) corresponding to the parallel data to a double density. Incidentally, X-ray penetration paths of opposite views are shifted in a channel direction so as not to overlap each other as shown in Fig. 43 and held in an interleave state.

If the view angles views are different by 180° in the projection data D0p(view, δ, j, i) corresponding to the parallel data, then they are brought to opposite views. Therefore, the projection data become easy to handle (e.g., it is easy to apply view weights of opposite views thereto). In contrast, even if view angles views are different by 180° in the case of the projection data D0f(view, δ, j, i) corresponding to the parallel data, they are not brought to opposite views except for a fan center. Therefore, their handling become complicated.

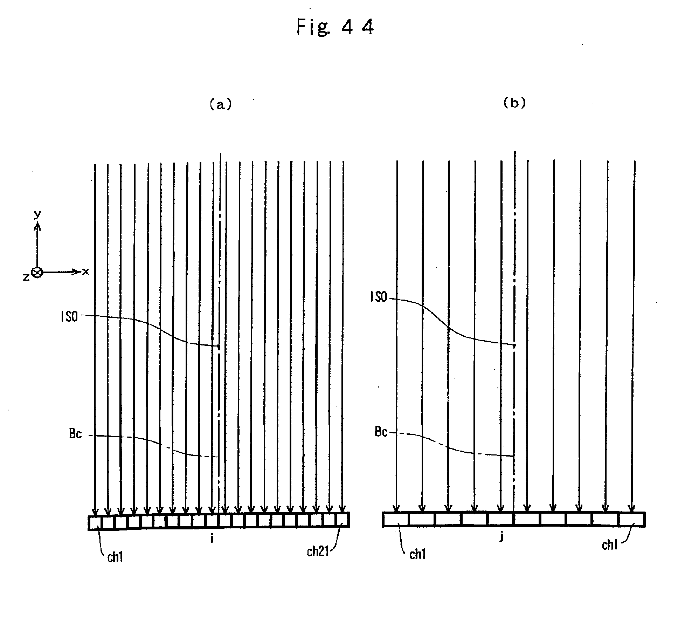

In Step F3, the projection data D0p(view, δ, i, j) corresponding to the parallel data are arranged according to an interpolation process as shown in Fig. 44 such that channel intervals are brought to equal intervals. Fig. 44(a) shows where opposite views are brought to a double density in combination. Fig. 44(b) shows where Step F2 is skipped.

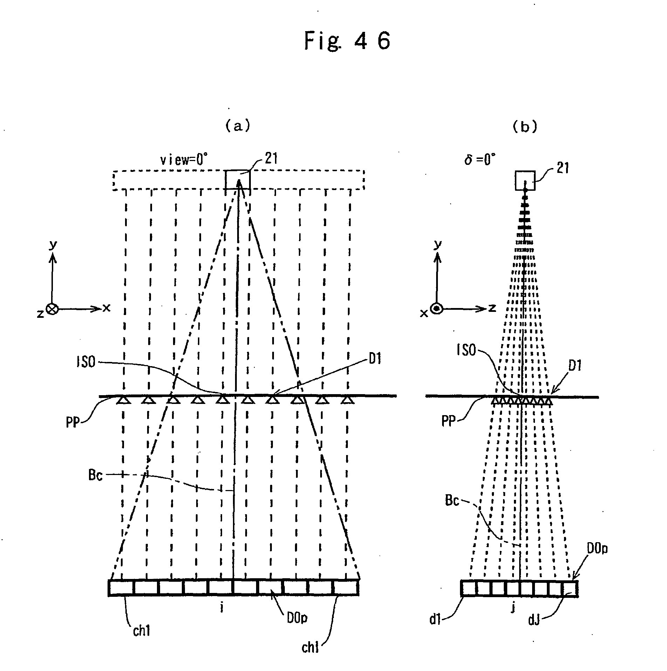

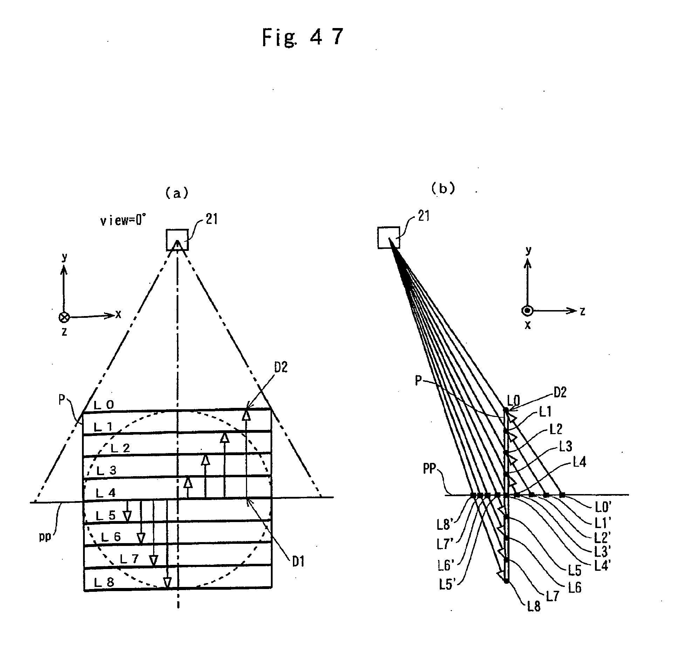

Fig. 45 is a detailed flowchart of the three-dimensional back projecting process (S16). In Step R11, plane-projected data D1(view, qt, pt) or data D1(view, Lm', pt) are determined from projection data D0p(view, δ, j, i) as shown in Fig. 46 in the same manner as described in the aforementioned example. Referring back to Fig. 45, in Step R12, backprojected pixel data D2(view, x, y) are determined from data D1(view, qt, pt) or data D1(view, Lm', pt) plane-projected onto the plane of projection as shown in Fig. 47 in the same manner as described in the aforementioned example.

In Step R13, in a manner similar to the description of the above example, views corresponding to 360° are added to backprojected pixel data D2(view, x, y) in association with pixels, or views corresponding to "180° + fan angle" are added thereto to obtain backprojected data D3(x, y).

In the respective examples referred to above, the following selections are enabled.

(1) Although the above example has explained the case in which "the number of lines"/"the number of pixels in the reconstruction area P in the direction orthogonal to the lines" has been set to = 9/512 ≈ 1/57, the number of the lines may be set to 8 to 256.

(2) Although the above example has been described under the assumption that 512 pixels are configured as the reconstruction area P, the present invention is applicable even to a 1024-pixel configuration and other number of pixels.

(3) Although the above example has been described assuming that the primary interpolation/extrapolation process is performed, a 0-order interpolation/extrapolation process (copy of most proximity data) or a secondary or more interpolation/extrapolation process (e.g., Hanning interpolation or Cubic interpolation) may be adopted.

(4) Although the above example has considered the interpolation using the two data D2 of the opposite view, helical interpolation using two data D2 of the same view may be adopted if an effective slice may become thick.

(5) Although such a view that the center axis Bc of the X-ray beam becomes parallel to the y axis, is assumed to be view = 0° in the above example, an arbitrary angle may be set as view = 0°.

(6) Although the medical X-ray CT apparatus has been supposed to be used in the above example, the present invention can be applied even to an industrial X-ray CT apparatus.

**Claims**

1. An image reconstructing method comprising the steps of, upon reconstructing an image using a multi-row detector having a plurality of detector sequences and on the basis of projection data D0 collected by a helical scan with a scan surface being tilted:

 effecting, on the projection data D0, a tilt correcting process for correcting variations every views, in positions of respective channels of the detector sequences with respect to a linear travel axis due to the inclination of the scan surface;
 then projecting respective pixels in an X-ray penetration direction along lines parallel to an X axis or a Y axis on a reconstruction plane (XY plane) to determine the corresponding projection data D0 and defining the same as backprojected pixel data D2 of respective pixels constituting the reconstruction plane; and
 adding the backprojected pixel data D2 of all views used in image reconstruction in association with the pixels to determine backprojected data D3.

2. An image reconstructing method comprising the steps of, upon reconstructing an image using a multi-row detector having a plurality of detector sequences and on the basis of projection data D0 collected by a helical scan with a scan surface being tilted:

 effecting, on the projection data D0, a tilt correcting process for correcting variations every views, in positions of respective channels of the detector sequences with respect to a linear travel axis due to the inclination of the scan surface;
 determining data D1 plane-projected onto the plane of projection on the basis of the tilt-corrected projection data D0;
 then projecting, in an X-ray penetration direction, the data D1 plane-projected on respective pixels constituting a plurality of lines which are parallel to an X axis or a Y axis on a reconstruction plane (XY plane) with plural pixel intervals defined thereamong and extend in the direction parallel to the projection plane to thereby determine backprojected pixel data D2 of the respective pixels constituting the lines on a reconstruction area; and
 interpolating among the plurality of lines to determine backprojected pixel data D2 of respective pixels among the lines on the reconstruction area; and
 adding the backprojected pixel data D2 of all views employed in image reconstruction in association with the pixels to determine backprojected data D3.

3. An image reconstructing method comprising the steps of, upon reconstructing an image using a multi-row detector having a plurality of detector sequences and on the basis of projection data D0 collected by a helical scan with a scan surface being tilted:

 effecting, on the projection data D0, a tilt correcting process for correcting variations every views, in positions of respective channels of the detector sequences with respect to a linear travel axis due to the inclination of the scan surface;
 determining data D1 plane-projected onto lines on a projection plane, corresponding to a plurality of lines which are placed on a reconstruction area with plural pixel intervals defined thereamong and extend in the direction parallel to the projection plane, on the basis of the tilt-corrected projection data D0;
 determining backprojected pixel data D2 of respective pixels on the reconstruction area on the basis of the plane-projected data D1 located on the lines on the projection plane; and
 adding the backprojected pixel data D2 of all views used in image reconstruction in association with the pixels to determine backprojected data D3.

4. An X-ray CT apparatus comprising:

 an X-ray tube;
 a multi-row detector opposite to the X-ray tube and having a plurality of detector sequences;
 a linear movement control device for relatively moving the X-ray tube and the multi-row detector linearly with a subject along a linear travel axis;
 a rotation control device for rotating at least one of the X-ray tube and the multi-row detector about a rotational axis;
 a tilt control device for tilting an angle of a scan surface formed by the rotation with respect to the linear travel axis to an angular slope other than 90°;

a scan control device for collecting projection data D0, using the multi-row detector and by a helical scan with a scan surface being tilted; and

an image reconstructing device for reconstructing an image, based on the projection data D0,

wherein the image reconstructing device includes,

a tilt correcting device for effecting a tilt correcting process for correcting variations every views, in positions of respective channels of the detector sequences with respect to the linear travel axis due to the inclination of the scan surface, on the projection data D0,

a backprojected pixel data calculating device for then projecting respective pixels in an X-ray penetration direction along lines parallel to an X axis or a Y axis on a reconstruction plane (XY plane) to determine the corresponding projection data D0 and thereby determining backprojected pixel data D2 of respective pixels constituting the reconstruction plane; and

a backprojected data calculating device for adding the backprojected pixel data D2 of all views used in image reconstruction in association with the pixels to determine backprojected data D3.

5.  The X-ray CT apparatus according to claim 4, further comprising a fan-para converting device for determining projection data D0p corresponding to parallel data from projection data D0f corresponding to fan data, wherein the scan control device collects the projection data D0f corresponding to the fan data, and the tilt correcting device effects a tilt correcting process on the projection data D0p corresponding to the parallel data.

6.  An X-ray CT apparatus comprising:

an X-ray tube;

a multi-row detector opposite to the X-ray tube and having a plurality of detector sequences;

a linear movement control device for relatively moving the X-ray tube and the multi-row detector linearly with a subject along a linear travel axis;

a rotation control device for rotating at least one of the X-ray tube and the multi-row detector about a rotational axis;

a tilt control device for tilting an angle of a scan surface formed by the rotation with respect to the linear travel axis to an angular slope other than $90°$;

a scan control device for collecting projection data D0, using the multi-row detector and by a helical scan with a scan surface being tilted; and

an image reconstructing device for reconstructing an image, based on the projection data D0,

wherein the image reconstructing device includes,

a tilt correcting device for effecting a tilt correcting process for correcting variations every views, in positions of respective channels of the detector sequences with respect to the linear travel axis due to the inclination of the scan surface, on the projection data D0,

a plane-projected data calculating device for determining data D1 plane-projected onto the plane of projection on the basis of the tilt-corrected projection data D0,

a backprojected pixel data calculating device for projecting, in an X-ray penetration direction, the plane-projected data D1 onto respective pixels constituting a plurality of lines which are parallel to an X axis or a Y axis on a reconstruction plane (XY plane) with plural pixel intervals defined thereamong and extend in the direction parallel to the projection plane to thereby determine backprojected pixel data D2 of the respective pixels constituting the lines on a reconstruction area, and interpolating among the plurality of lines to determine backprojected pixel data D2 of respective pixels among the lines on the reconstruction area, and

a backprojected data calculating device for adding the backprojected pixel data D2 of all views employed in image reconstruction in association with the pixels to determine backprojected data D3.

7.  An X-ray CT apparatus comprising:

an X-ray tube;

a multi-row detector opposite to the X-ray tube and having a plurality of detector sequences;

a linear movement control device for relatively moving the X-ray tube and the multi-row detector linearly with a subject along a linear travel axis;

a rotation control device for rotating at least one of the X-ray tube and the multi-row detector about a rotational axis;

a tilt control device for tilting an angle of a scan surface formed by the rotation with respect to the linear travel axis to an angular slope other than 90°;
a scan control device for collecting projection data D0, using the multi-row detector and by a helical scan with a scan surface being tilted; and
an image reconstructing device for reconstructing an image, based on the projection data D0,

wherein the image reconstructing device includes,
tilt correcting device for effecting a tilt correcting process for correcting variations every views, in positions of respective channels of the detector sequences with respect to the linear travel axis due to the inclination of the scan surface, on the projection data D0,
a plane-projected data calculating device for determining data D1 plane-projected onto lines on a projection plane, corresponding to a plurality of lines which are placed on a reconstruction area with plural pixel intervals defined thereamong and extend in the direction parallel to the projection plane, on the basis of the tilt-corrected projection data D0,
a backprojected pixel data calculating device for determining backprojected pixel data D2 of respective pixels on the reconstruction area on the basis of the plane-projected data D1, and
a backprojected data calculating device for adding the backprojected pixel data D2 of all views used in image reconstruction in association with the pixels to determine backprojected data D3.

8. The X-ray CT apparatus according to claim 6 or 7, wherein the number of the lines ranges from 1/64 to 1/2 of the number of the pixels in the reconstruction area as viewed in the direction orthogonal to the lines.

9. The X-ray CT apparatus according to any of claims 4 to 8, wherein when the direction orthogonal to a rotating plane of the X-ray tube or the multi-row detector is defined as a z direction, the direction of a center axis of an X-ray beam at view = 0° is defined as a y direction, and the direction orthogonal to the z direction and the y direction is defined as an x direction, the plane-projected data calculating device sets an xz plane passing through the center of rotation as the projection plane in -45° ≤view< 45° or a view angular range containing even a periphery with this view as a principal part, and 135° ≤view< 225° or a view angular range containing even a periphery with this view as a principal part, and sets a yz plane passing through the center of rotation as the projection plane in 45° ≤view< 135° or a view angular range containing even a periphery with this view as a principal part, and 225° ≤view< 315° or a view angular range containing even a periphery with this view as a principal part.

10. The X-ray CT apparatus according to any of claims 6 to 9, wherein the plane-projected data calculating device determines one plane-projected data D1 from the plurality of projection data D0 by an interpolation/extrapolation process.

Fig. 1

(a)

(b)

Fig. 2

```
        ┌─────────────┐
        (   START     )
        └──────┬──────┘
               │              S0
        ┌──────┴──────┐
        │ Data acquisition │
        └──────┬──────┘
               │              S1
        ┌──────┴──────┐
        │ Pre-treatment │
        └──────┬──────┘
               │              S12
        ┌──────┴──────┐
        │   Fan-para    │
        │converting process│
        └──────┬──────┘
               │              S2
        ┌──────┴──────┐
        │ Tilt correcting │
        │    process    │
        └──────┬──────┘
               │              S3
        ┌──────┴──────┐
        │ Filter process │
        └──────┬──────┘
               │              S4
    ┌──────────┴──────────┐
    │  Three-dimensional   │
    │back projection process│
    └──────────┬──────────┘
               │              S5
        ┌──────┴──────┐
        │ Post-treatment │
        └──────┬──────┘
               │
        ┌──────┴──────┐
        (    END      )
        └─────────────┘
```

# Fig. 3

```
        ┌──────────────────────┐
        │  Start tilt correcting│  S2
        │       process         │
        └──────────────────────┘
                   │                    T1
        ┌──────────────────────┐
        │ Move data position in channel│
        │ direction by shift in position│
        └──────────────────────┘
                   │                    T2
        ┌──────────────────────┐
        │     Cut out data in   │
        │  predetermined range  │
        └──────────────────────┘
                   │                    T3
        ┌──────────────────────┐
        │  Add air data and arrange│
        │       data range      │
        └──────────────────────┘
                   │                    T4
        ┌──────────────────────┐
        │ Convert data with channel position│
        │   of all views being aligned│
        └──────────────────────┘
                   │
        ┌──────────────────────┐
        │          END          │
        └──────────────────────┘
```

# Fig. 4

# Fig. 5

# Fig. 6

Position of ith channel with respect to linear travel axis

# Fig. 7

Fig. 8

Fig. 9

Fig. 1 0

# Fig. 1 1

Channel number
i

View number

pvn

VWN

Air_Data

Air_Data

1_pvn_src[i]

Fig. 1 2

Fig. 1 3

# Fig. 1 4

Fig. 1 5

Start tilt correcting process

Cut out data in predetermined range `T11`

Add air data and arrange data range `T12`

Convert to data aligned with channel positions of all views being aligne `T13`

END

Fig. 1 6

Start three-dimensional back projecting process `S4`

Obtain plane-projected data D1 (view, qt, pt) from projection data D0 (view, δ, j, i) `R1`

Obtain backprojected pixel data D2 (view, x, y) from plane-projected data D1 (view, qt, pt) `R2`

Determine backprojected data directly from projection data D0 `R12`

Add backprojected pixel data D2 (view, x, y) over all views in association with pixels to obtain backprojected data D3 (x, y) `R3`

END

# Fig. 17

(a)

(b)

Plane of Projection
pp

Center of rotation
ISO

Center axis
Bc

ch1

chI

24

Plane of projection
pp

Bc

Center of rotation
ISO

d1
First detector
sequence

Jth detector sequence
dJ

24

(c)

pp

ISO

view=0°

pt

0

pe

j

j

δ = 0°

1

Z0

D1' (0, 0, j, pt)
Plane-projected original data

# Fig. 18

(a)

(b)

(c)

# Fig. 19

view=0°

pp

Relative angle difference δ

0    pt    Pe
J
j↑
1
720°
D1' (0, 720, j, pt)

0    pt    Pe
J
j↑
1
360°
D1' (0, 360, j, pt)

0    pt    Pe
J
j↑
1
0°
Z0
D1' (0, 0, j, pt)

z
↑
⊙ →x
y

# Fig. 20

view=0°

0    pt    Pe
Qe

qt↑

0
Z0
D1 (0, qt, pt)
Plane-projected data

z
↑
⊙ →x
y

# Fig. 2 1

view=30°

Relative angle difference δ

pp

0 → pt → Pe

J ... 720°

D1' (30, 720, j, pt)

0 → pt → Pe

J ... 360°

D1' (30, 360, j, pt)

0 → pt → Pe

J ... 0°

D1' (30, 0, j, pt)

Z30

z, x, y

# Fig. 2 2

view=30°

pt → 

Qe  0         Pe

qt ↑

0

Z30

D1 (30, qt, pt)
Plane-projected data

z, x, y

# Fig. 2 3

(a)

(b)

(c)

D1' (90, δ, j, pt)
Plane-projected
original data

# Fig. 2 4

(a) Lookup table (for plane projection)   (b) Lookup table (for plane projection)

### (a) — 31

| view=45° −Δview | | | | |
| view=··· | | | | |
| view=0° | | | | |
| view=··· | | | | |
| view=−45° | | | | |

| j | pt | i | k1 | k2 |
|---|----|---|----|----|
| 1 | 0 | | | |
|   | 1 | | | |
|   | 2 | | | |
|   | ⋮ | | | |
|   | Pe | | | |
| 2 | 0 | | | |
|   | 1 | | | |
|   | 2 | | | |
|   | ⋮ | | | |
|   | Pe | | | |
| ⋮ | ⋮ | | | |
| J | 0 | | | |
|   | 1 | | | |
|   | 2 | | | |
|   | ⋮ | | | |
|   | Pe | | | |

### (b) — 31'

| view=45° −Δview | | | | | |
| view=··· | | | | | |
| view=0° | | | | | |
| view=··· | | | | | |
| view=−45° | | | | | |

| j | pt | i | k1 | k2 | k3 |
|---|----|---|----|----|----|
| 1 | 0 | | | | |
|   | 1 | | | | |
|   | 2 | | | | |
|   | ⋮ | | | | |
|   | Pe | | | | |
| 2 | 0 | | | | |
|   | 1 | | | | |
|   | 2 | | | | |
|   | ⋮ | | | | |
|   | Pe | | | | |
| ⋮ | ⋮ | | | | |
| J | 0 | | | | |
|   | 1 | | | | |
|   | 2 | | | | |
|   | ⋮ | | | | |
|   | Pe | | | | |

# Fig. 2 5

Fig. 2 6

(a)

(b)

# Fig. 2 7

(a)

(b)

(c)

(d)

# Fig. 2 8

Lookup table (for back projection)

32

| y | R(y)_a | str_x | str_qt | $\triangle$qt | $\triangle$pt | n(y) |
|---|---|---|---|---|---|---|
| 0 | | | | | | |
| Ye/8 | | | | | | |
| 2Ye/8 | | | | | | |
| 3Ye/8 | | | | | | |
| 4Ye/8 | | | | | | |
| 5Ye/8 | | | | | | |
| 6Ye/8 | | | | | | |
| 7Ye/8 | | | | | | |
| Ye | | | | | | |

view=45° $-\triangle$view

view=

view=0°

view=

view=-45°

## Fig. 29

-45° ≦view<45°    135° ≦view<225°

$R(y)\_a = (r0\_0a/r0\_1a)^2$

$D2(view, x, y)\_a = R(y)\_a \times D1(view, str\_qt+(x-str\_x)\,\Delta qt$
$, (x-str\_x)\,\Delta pt)$

# Fig. 3 0

(a)

P      view=0°

L 0
L 1
L 2
L 3
L 4
L 5
L 6
L 7
L 8

D2 (0, x, y) _a

(b)

P      Opposite view

L 0
L 1
L 2
L 3
L 4
L 5
L 6
L 7
L 8

D2 (0, x, y) _b

# Fig. 3 1

(a)

P      view=0°

L 0
L 1
L 2
L 3
L 4
L 5
L 6
L 7
L 8

D2 (0, x, y)

(b)

P      view=0°

D2 (0, x, y)

Fig. 3 2

Fig. 3 3

(a)

(b)

# Fig. 3 4

view=0°

L8'
L7'
L6'
L5'
L4'
L3'
L2'
L1'
L0'

0    Pt→    Pe

PP

z
↑
⊙—→x
y

# Fig. 35

# Fig. 3 6

Lookup table (for back projection)

32'

| view=45° −Δview | | | | | | |
|---|---|---|---|---|---|---|

| view= | | | | | | |
|---|---|---|---|---|---|---|

| view=0° | | | | | | |
|---|---|---|---|---|---|---|

| view= | | | | | | |
|---|---|---|---|---|---|---|

view=−45°

| y | km | km+1 | S (y) | str_x | Δpt | n (y) |
|---|---|---|---|---|---|---|
| 0 | 1 | 0 | | | | |
| | | | | | | |
| 0.6Ye/8 | 0.4 | 0.6 | | | | |
| | | | | | | |
| Ye/8 | 1 | 0 | | | | |
| | | | | | | |
| | | | | | | |
| | | | | | | |
| Ye | | | | | | |

# Fig. 3 7

Fig. 3 8

Fig. 3 9

```
        ┌─────────────┐
        │    START    │
        └──────┬──────┘
               │          ┌S11
        ┌──────┴──────┐
        │    Data     │
        │ acquisition │
        └──────┬──────┘
               │          ┌S12
        ┌──────┴──────┐
        │Pre-treatment│
        └──────┬──────┘
               │          ┌S13
        ┌──────┴──────┐
        │Fan-para conberting│
        │   process   │
        └──────┬──────┘
               │          ┌S14
        ┌──────┴──────┐
        │Tilt correcting│
        │   process   │
        └──────┬──────┘
               │          ┌S15
        ┌──────┴──────┐
        │Filter process│
        └──────┬──────┘
               │          ┌S16
        ┌──────┴──────┐
        │Three-dimensional│
        │back projecting process│
        └──────┬──────┘
               │          ┌S17
        ┌──────┴──────┐
        │Post-treatment│
        └──────┬──────┘
               │
        ┌──────┴──────┐
        │     END     │
        └─────────────┘
```

# Fig. 4 0

S13 ( Start fan-para converting process )

F1

Create projection data D0p(view,d,j,i) corresponding
to parallel data from projection data D0f(view,d,i,j)
corresponding to fan data

F2

When helical pitch is small, combine opposite views
to arrange projection data D0p(view,d,i,j)
corresponding to parallel data

F3

Arrange projection data D0p(view,d,i,j) corresponding
to parallel data such that channel intervals reach
equal intervals

( END )

Fig. 4 1

(b)

Projection data corresponding to parallel data
D0p(view, δ, i, j)

δ=0

view

j

i

(a)

Projection data corresponding to fan data
D0f(view, δ, i, j)

δ=0

view

j

i

Fig. 42

(a)

(b)

Fig. 4 3

Fig. 4 4

(a)

(b)

# Fig. 4 5

```
        ┌─────────────────────────────┐
   S16  (  START three-dimensional     )
        (   back projecting process    )
        └─────────────────────────────┘
                      │                        R11
        ┌─────────────────────────────┐
        │  Obtain plane-projected data D1(view,qt,pt) or  │
        │  data D1(view,Lm',pt) from projection data      │
        │              D0p(view,d,i,j)                     │
        └─────────────────────────────┘
                      │                        R12
        ┌─────────────────────────────┐
        │  Obtain backprojected pixel data D2(view,x,y)   │
        │  from plane-projected data D1(view,qt,pt) or     │
        │              data D1(view,Lm',pt)                │
        └─────────────────────────────┘
                      │                        R13
        ┌─────────────────────────────┐
        │  Add backprojected pixel data D2(view,x,y)      │
        │  over all views in association with pixels to    │
        │      obtain backprojected data D3(x,y)           │
        └─────────────────────────────┘
                      │
               ┌─────────────┐
               (     END     )
               └─────────────┘
```

Fig. 46

(a)

view=0°   21

(b)

δ=0°   21

Fig. 47

(a)

(b)